(19) Europäisches Patentamt
European Patent Office
Office européen des brevets

(11) Veröffentlichungsnummer: 0 255 028
A2

(12) # EUROPÄISCHE PATENTANMELDUNG

(21) Anmeldenummer: 87110590.4

(22) Anmeldetag: 22.07.87

(51) Int. Cl.³: C 07 D 487/04
A 61 K 31/55
//(C07D487/04, 249:00, 243:00),
(C07D487/04, 243:00, 235:00)

(30) Priorität: 25.07.86 DE 3625197

(43) Veröffentlichungstag der Anmeldung:
03.02.88 Patentblatt 88/5

(84) Benannte Vertragsstaaten:
AT BE CH DE ES FR GB GR IT LI LU NL SE

(71) Anmelder: BOEHRINGER INGELHEIM KG

D-6507 Ingelheim am Rhein(DE)

(84) Benannte Vertragsstaaten:
BE CH DE ES FR GR IT LI LU NL SE AT

(71) Anmelder: Boehringer Ingelheim International G.m.b.H

D-6507 Ingelheim am Rhein(DE)

(84) Benannte Vertragsstaaten:
GB

(72) Erfinder: Walther, Gerhard, Dr. Dipl.-Chem.
Pfarrer-Hebener Strasse 37
D-6530 Bingen(DE)

(72) Erfinder: Harreus, Albrecht, Dr. Dipl.-Chem.
Sandstrasse 1
D-6507 Ingelheim am Rhein(DE)

(72) Erfinder: Weber, Karl-Heinz, Dr. Dipl.-Chem.
Kaiser-Karl-Strasse 11
D-6535 Gau-Algesheim(DE)

(72) Erfinder: Stransky, Werner, Dr. Dipl.-Chem.
Im Hippel 24
D-6535 Gau-Algesheim(DE)

(72) Erfinder: Muacevic, Gojko, Dr.
In der Dörrwiese 13
D-6507 Ingelheim am Rhein(DE)

(72) Erfinder: Casals Stenzel, Jorge, Dr.
Hirsch-Gereuth-Strasse 76
D-8000 München 70(DE)

(72) Erfinder: Bechtel, Wolf-Dietrich, Dr.
Mühlstrasse 3
D-6531 Appenheim(DE)

(54) **Neue 1,4-Benzodiazepine, ihre Herstellung und Verwendung.**

(57) Die neuen 1,4-Benzodiazepine der Formel Ia bzw. Ib die in der Beschreibung dargestellt werden, können nach üblichen Methoden hergestellt werden und eignen sich als Wirkstoffe für Arzneimittel.

EP 0 255 028 A2

Die Erfindung betrifft neue 1,4-Benzodiazepine, ihre Herstellung nach üblichen Methoden und ihre Verwendung als Arzneistoffe und als Zwischenprodukte.

Die neuen 1,4-Benzodiazepine entsprechen den Formeln

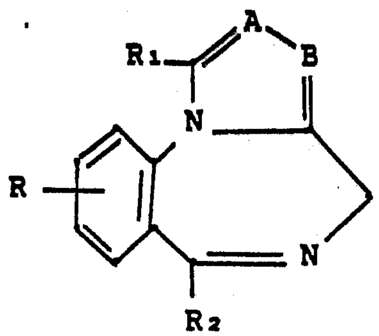

(Ia)

oder

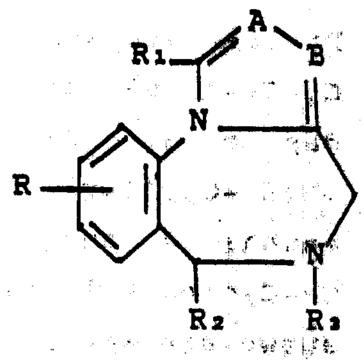

(Ib),

worin

R für eine der Gruppen

(a) $-O-X-CO-OR_4$

(b) $-O-X-CO-R_5$

(c) $-O-Y-R_6$

(d) $-Z-CO-OR_4$

(e) $-Z-CO-R_5$

(f) 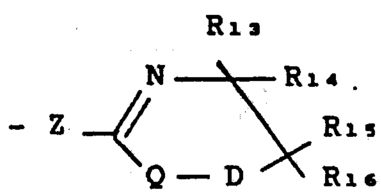 ;

(g) -O-X 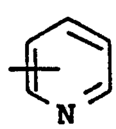

$R_1$ für Wasserstoff; für $C_1$-$C_4$-Alkyl oder für $C_3$-$C_6$-Cycloalkyl; oder, falls R die Gruppe (d), (e) oder (f) bedeutet, auch für $C_1$-$C_4$-Alkoxy oder Halogen;

$R_2$ für Phenyl, für ein- oder mehrfach durch Methyl, Halogen, Nitro, Trifluormethyl oder, falls R die

Gruppe (d), (e) oder (f) bedeutet, auch durch Methoxy substituiertes Phenyl; oder für α-Pyridyl;

$R_3$ für Wasserstoff oder für $C_1$-$C_4$-Alkyl;

$R_4$ für einen durch eine Mono- oder Di-($C_1$-$C_3$-alkyl)aminogruppe substituierte $C_1$-$C_6$-Alkylgruppe; für $C_3$-$C_7$-Cycloalkyl, das im Falle des 5- oder 6-Rings auch eine >N($C_1$-$C_4$-Alkyl)-Gruppe als Ringglied enthalten kann und das im Fall des $C_7$-Cycloalkyls auch eine >$NCH_3$-Brücke aufweisen kann; oder, falls R die Gruppe (a) oder (d) bedeutet, auch für Wasserstoff oder $C_1$-$C_6$-Alkyl;

$R_5$ für eine der Gruppen

(a) 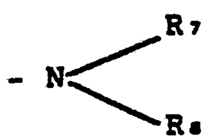 oder

(b) 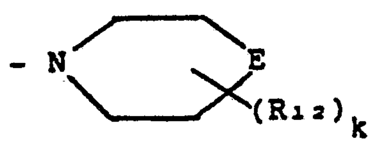 ;

$R_6$ für eine der Gruppen

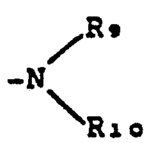 oder 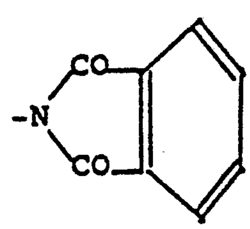 oder 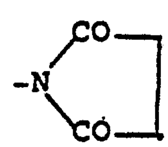 ;

oder die unter R (f) aufgeführte Gruppe, worin Z eine Einfachbindung darstellt;

$R_7$ und $R_8$ (unabhängig voneinander) für Wasserstoff; für $C_1$-$C_6$-Alkyl, Allyl oder Propargyl; und, falls $R_7$ Wasserstoff oder Methyl bedeutet, $R_8$ auch für $C_3$-$C_6$-Cycloalkyl, Aryl, Aralkyl oder eine der Gruppen

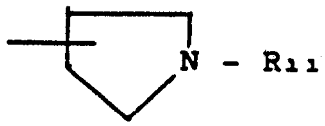

oder 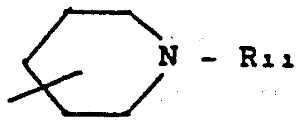

oder 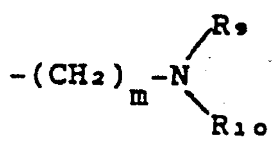 ;

oder,
falls R die Gruppe (b), darstellt, auch für eine der Gruppen

$-CHR_3-COO(C_1-C_4\text{-Alkyl})$ oder

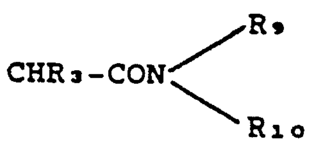 ;

$R_9$ und $R_{10}$ (unabhängig voneinander) für Wasserstoff; für $C_1$-$C_6$-Alkyl; außerdem (zusammen mit dem Stickstoffatom der Gruppe $-NR_9R_{10}$) für einen gegebenenfalls ein- bis vierfach methylsubstituierten gesättigten fünf- oder einen sechsgliedrigen Ring, wobei dieser auch ein weiteres Heteroatom (O, S, $NR_3$) enthalten kann;

$R_{11}$ für $C_1$-$C_4$-Alkyl, Aryl oder Aralkyl;

$R_{12}$ für Wasserstoff oder Methyl, einer der Reste $R_{12}$ gegebenenfalls auch für $-CO-O(C_1-C_6-Alkyl)$,

$R_{13}$ für Wasserstoff; für ein gegebenenfalls hydroxy- oder aminosubstituiertes $C_1-C_6$-Alkyl; für eine $CO-O(C_1-C_4-Alkyl)$- oder eine $CO-N(C_1-C_4-Alkyl)_2$-Gruppe;

$R_{14}$, $R_{15}$, $R_{16}$, $R_{17}$ und $R_{18}$ (unabhängig voneinander) für Wasserstoff; für ein gegebenenfalls hydroxy- oder aminosubstituiertes $C_1-C_6$-Alkyl; oder für Phenyl;

A und B (unabhängig voneinander) für N, CH oder $C-CH_3$, aber nicht beide für CH oder $C-CH_3$,

D für $(CR_{17}R_{18})_n$;

Q für O, S, NH oder $N(C_1-C_6-Alkyl)$;

E für O, S, $NR_{11}$, $CH_2$ oder eine Einfachbindung;

X für $C_1-C_6$-Alkylen;

Y für $C_2-C_6$-Alkylen;

Z für $C_1-C_6$-Alkylen, eine Einfachbindung oder die Gruppe $OCH_2$, wobei der Sauerstoff an den Benzolring gebunden ist;

k für 1, 2, 3 oder 4;

m für 2 oder 3;

n für O, 1, oder 2;

stehen kann,

gegebenenfalls in Form der Racemate, der Enantiomeren, Diastereomeren und ihrer Gemische; jeweils als freie Basen oder als Säureadditionssalze, gegebenenfalls auch als quaternisierte Verbindungen.

In den obigen Definitionen und im folgenden bedeutet "Aryl" einen gegebenenfalls ein- oder mehrfach substituierten Phenyl-, Pyridyl-, Thienyl- oder Furylrest, wobei als Substituenten ein oder mehrere Atome aus der Gruppe Halogen, Methyl, Methoxy, Trifluormethyl vorliegen können. "Aralkyl" ist eine Gruppe Aryl-($C_1$-$C_3$-alkyl), worin Aryl die obige Bedeutung hat. Die Kohlenstoffketten in den Alkyl-, Alkoxy- und Alkylenresten, auch soweit diese Bestandteile anderer Reste sind, können geradkettig oder verzweigt sein. Bei Mehrfachsubstitution können gleiche oder verschiedene Substituenten nebeneinander vorliegen.

Unter "Halogen" werden Fluor, Chlor, Brom und Jod verstanden; bevorzugt sind Fluor, Chlor und Brom, insbesondere Chlor und Brom.

Von den Alkylresten (auch soweit sie Bestandteile anderer Reste sind) sind Methyl und Ethyl besonders wichtig, vor allem Methyl.

Im Rahmen der Definitionen für die einzelnen Symbole sind die folgenden Bedeutungen hervorzuheben:

A und B Stickstoff oder A CH-Gruppe und B Stickstoff;

$R_1$: Methyl, Methoxy und Cyclopropyl;

$R_2$: 2-Chlorphenyl;

R: vorzugsweise in 8-Stellung mit dem Ringsystem verknüpft;

für die Gruppen (a) bis (g) gilt vor allem:

bei (a): X gleich $C_1$-$C_3$-Alkylen
$R_4$ in der obigen Bedeutung, ausgenommen Wasserstoff und $C_1$-$C_6$-Alkyl;

bei (b): X gleich $C_1$-$C_3$-Alkylen,
$R_5$ gleich $-NR_7R_8$ mit $R_7$, $R_8$ gleich Wasserstoff oder $C_1$-$C_4$-Alkyl;
oder
$R_7$ gleich H und
$R_8$ gleich
$C_2$-$C_3$-Alkylsubstituiert durch N-Morpholino oder 2,6-Dimethylmorpholino;
oder
$R_5$ gleich

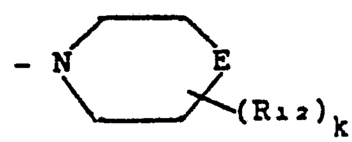

worin
E gleich O, N-Methyl, $CH_2$;
dabei ist im Falle E gleich O, $R_{12}$ bevorzugt Methyl und k gleich 2, wobei die Methylgruppen sich in 2- und 6-Stellung befinden, während für E gleich $CH_2$ $R_{12}$ vor allem $COOC_2H_5$ ist;

bei (c): Y gleich $C_1$-$C_3$-Alkylen,
$R_6$ Phthalimido oder $NR_9R_{10}$ und
$R_9$/$R_{10}$ gleich H oder

$C_1$-$C_4$-Alkyl oder $NR_9R_{10}$ gleich Morpholino, gegebenenfalls methylsubstituiert;

bei (d): Z ist bevorzugt eine Einfachbindung,
$R_4$ in der obigen Bedeutung, ausgenommen Wasserstoff und $C_1$-$C_6$-Alkyl;

bei (e): Z ist bevorzugt eine Einfachbindung,
$R_5$ $NR_7R_8$ mit $R_7$ gleich H,

$R_8$ $-CH_2-CH_2-$ 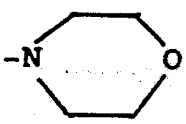 oder

$-C_1-C_6$-Alkyl;

bei (f) Z ist bevorzugt eine Einfachbindung, ebenso D (n = O), $R_{13}/R_{14}$ Methyl, $R_{15}/R_{16}$ Wasserstoff,

bei (g) X ist bevorzugt $-CH_2-$ oder $-CH_2-CH_2-$

Die neuen Verbindungen können nach an sich bekannten Verfahren hergestellt werden.

1. Zur Herstellung von Verbindungen der Formel Ia, worin R die Gruppe
   (a): $-OX-CO-OR_4$ bedeutet, werden die Verbindungen der Formel

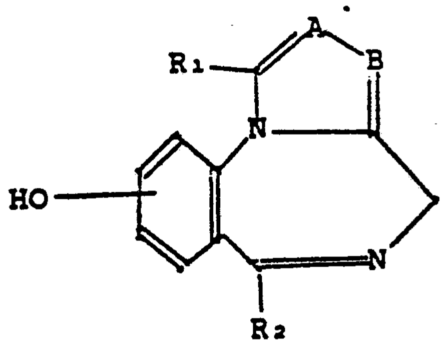

(II)

worin A, B, $R_1$ und $R_2$ die obige Bedeutung haben, mit Verbindungen der Formel

Halogen - X - $COOR_4$ (III)

(Halogen gleich Brom oder Chlor, X und $R_4$ wie oben definiert) umgesetzt.

Die Umsetzung erfolgt, z.B. in an sich bekannter Weise, in Gegenwart einer Base, z.B. Kaliumcarbonat, gegebenenfalls unter Zusatz katalytischer Mengen Natrium- oder Kaliumjodid, in einem organischen Lösungsmittel, wie Aceton, Ethylmethylketon, Dimethylformamid etc. Die angewandten Reaktionstemperaturen liegen in Abhängigkeit vom Lösungsmittel zwischen Raumtemperatur und der Siedetemperatur des verwendeten Lösungsmittels.

Die Verbindungen der Formel II sind bekannt oder können nach herkömmlichen Methoden erhalten werden.

Die Verbindungen der allgemeinen Formel II werden z.B. aus Verbindungen der allgemeinen Formel

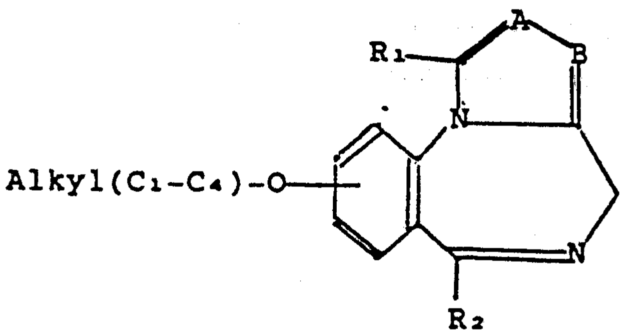

(IV)

worin A, B, $R_1$ und $R_2$ die obige Bedeutung haben, durch Ätherspaltung, z.B. mit Pyridin-hydrochlorid oder Bromwasserstoffsäure, dargestellt. Die Umsetzung erfolgt in an sich bekannter Weise mit Pyridinhydrochlorid in der Schmelze bei 100-200°C, bevorzugt bei 150-180°C oder mit Bromwasserstoffsäure, bevorzugt mit 60%iger wäßriger Bromwasserstoffsäure, in der Siedehitze.

Die Verbindungen der allgemeinen Formel IV sind teilweise bekannt oder können in Analogie zu bekannten Verfahren hergestellt werden.

Ausgehend von den Carbonsäureestern V der allgemeinen Formel Ia mit R = -Z-COOR' oder R = -O-X-COOR' (R' = Niederalkyl) können die entsprechenden Carbonsäuren durch Verseifung, z.B. mit NaOH oder KOH in Ethanol, erhalten werden

Diese Carbonsäureester V und die entsprechenden Säuren sind auch wertvolle Ausgangsverbindungen zur Einführung weiterer funktioneller Gruppen.

2. Zur Herstellung der Verbindungen der Formel Ia, worin R für die Gruppe (b): $-O-X-CO-R_5$ oder (e): $-Z-CO-R_5$ steht, werden die entsprechenden Carbonsäuren mit den Gruppen $-O-X-COOH$ bzw. $-Z-COOH$ oder reaktionsfähige Derivate davon mit den entsprechenden Aminen der Formel

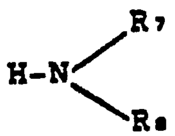 bzw. 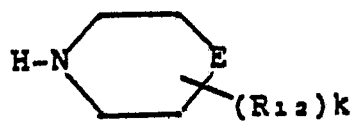

(VI) (VII)

oder mit Ammoniak umgesetzt.

Bevorzugt ist die Überführung in ein Carbonsäurechlorid oder Säureanhydrid oder die Umsetzung der Säure in Gegenwart von Carbonyldiimidazol, Sulfonyldiimidazol, Cyclohexylcarbodiimid.

Die Umsetzung der freien Säure mit dem Amin erfolgt in Gegenwart eines Carbodiimids, beispielsweise von Cyclohexylcarbodiimid, Carbonyldiimidazols oder Sulfonyldiimidazols in einem inerten Lösungsmittel wie Dimethylformamid, Tetrahydrofuran, Dioxan oder halogenierten Kohlenwasserstoff bei Temperaturen zwischen 0°C und dem Siedepunkt des Reaktionsgemisches.

Bei der Reaktion des Amins mit einem Säurehalogenid oder Säureanhydrid wird das Amin in einem inerten Lösungsmittel, beispielsweise Dimethylformamid, Tetrahydrofuran, Dioxan oder einem geeigneten Kohlenwasserstoff wie Toluol bei Temperaturen zwischen

Raumtemperatur und dem Siedepunkt des Reaktionsgemisches mit dem Säurehalogenid oder dem Säureanhydrid umgesetzt, wobei gegebenenfalls ein säurebindendes Mittel wie Natriumcarbonat, Natriumbicarbonat oder eine tertiäre organische Base, beispielsweise Pyridin oder Triethylamin, zugegeben wird.

Falls es sich bei dem Amin um eine Flüssigkeit handelt, kann die Umsetzung auch in einem Überschuß des Amins ohne zusätzliches Lösungsmittel erfolgen.

Das Säurehalogenid bzw. Säureanhydrid erhält man aus der freien Säure in üblicher Weise, z.B. durch Reaktion der Säure mit einem Thionylhalogenid bzw. durch Umsetzung eines Alkalisalzes der Säure mit Acetylchlorid oder Chlorameisensäurechlorid.

Nach den gleichen Methoden können aus den Carbonsäuren auch die erfindungsgemäßen Carbonsäureester der Formel V hergestellt werden. Dabei können die Alkohole auch in Form der Alkoholate eingesetzt werden.

3. Zur Herstellung der Verbindungen der Formel Ia, worin R die Gruppe
(c): $- O - X - R_6$ bedeutet, werden Verbindungen der Formel II mit Verbindungen der Formel

$$\text{Halogen} - Y - R_6 \qquad \text{(VIII)}$$

worin Y und $R_6$ die obige Bedeutung haben und Halogen bevorzugt Chlor ist, umgesetzt.

Die Umsetzung erfolgt bevorzugt in Gegenwart eines säurebindenden Mittels, z.B. Kaliumcarbonat, in einem

geeigneten organischen Lösungsmittel, z.B. Aceton, Ethylmethylketon oder DMF.

Die Verbindungen der Formel VIII können auch in Form ihrer Säureadditionssalze eingesetzt werden. Auch können die Verbindungen der Formel VIII statt Halogen auch eine andere übliche Austrittsgruppe enthalten.

Die Verbindungen der Formel VIII sind bekannt oder nach üblichen Methoden erhältlich.

4. Zur Herstellung von Verbindungen der Formel Ia, R die Gruppe (c): - O - Y - $R_6$ und $R_6$ $NH_2$ bedeutet, werden entsprechende Phthalimidoverbindungen einer Hydrazinolyse, z.B. in Ethanol, unterworfen.

5. Zur Herstellung von Verbindungen der Formel Ia, worin R die Gruppe (f):

(f) 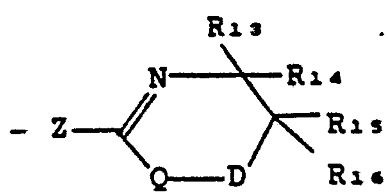 (IX)

bedeutet, oder solcher Verbindungen der Gruppe R(c), in denen $R_6$ für (IX) steht,
setzt man Carbonsäuren der Formel

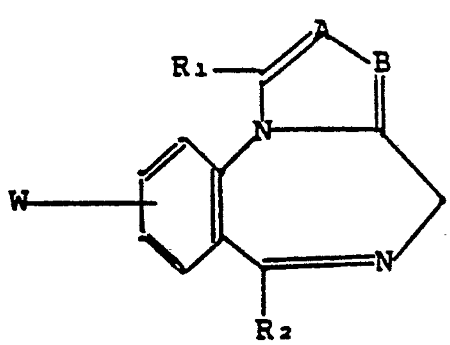 (X)

worin A, B, $R_1$ und $R_2$ die obige Bedeutung haben und W für -Z-COOH oder -O-X-COOH steht (Z und X wie oben definiert), mit einem bisfunktionalisierten Amin, z.B. einem Aminoalkohol, einem Aminomercaptan oder einem Diamin in Gegenwart von Triphenylphosphin, Tetrachlorkohlenstoff und einer tertiären organischen Base in Acetonitril um.

Die Reaktion erfolgt im Temperaturbereich zwischen 0°C und dem Siedepunkt der Reaktionsmischung, bevorzugt zwischen 0°C und Raumtemperatur.

6. Verbindungen der Formel Ia, worin R die Gruppe IX oder (c) in der entsprechenden Bedeutung ist und Q Sauerstoff oder Schwefel bedeutet, erhält man auch aus den entsprechenden hydroxyfunktionalisierten Carbonsäureamiden durch Ringschlußreaktion mit Thionylchlorid in einem organischen Lösungsmittel, z.B. Methylenchlorid und gegebenenfalls anschließende Schwefelung zum Thiazolin.

Dies kann mit Phosphorpentasulfid oder Lawesson-Reagenz® erfolgen.

7. Zur Herstellung von Verbindungen der Formel Ia, worin R die Gruppe (f) oder die Gruppe (c), worin $R_6$ für einen Rest der Formel IX steht, bedeutet, wobei Q für NH oder N($C_1$-$C_6$-Alkyl) steht, werden Verbindungen der Formel Ia mit R in der Bedeutung Z-CN oder -O-Y-CN über das Imidoethylesterhydrochlorid durch Reaktion mit einem entsprechenden Diamin umgesetzt (Pinner-Reaktion).

Die Bildung des Imidoethylesterhydrochlorids erfolgt

durch Behandlung des Nitrils mit einem Überschuß an ethanolischer Salzsäure. Das entstandene kristalline Rohprodukt wird in Ethanol mit dem Diamin (z.B. Ethylendiamin) zuerst unter Eiskühlung, dann unter Rückflußbedingungen umgesetzt. Dessen Aminofunktion kann im Fall von N-H nach bekannten Methoden alkyliert werden

Verbindungen der Formel Ia mit R gleich -Z-CN oder -O-Y-CN erhält man z.B. aus den entsprechenden Carbonsäureamiden durch Reaktion mit Phosphoroxychlorid in einem inerten organischen Lösungsmittel, z.B. Dichlorethan unter Rückfluß.

8. Zur Herstellung solcher Verbindungen der Formel Ia, in denen $R_1$ Halogen bedeutet, können auch entsprechende Verbindungen mit $R_1$ gleich Wasserstoff in üblicher Weise halogeniert werden.

9. Zur Herstellung von Verbindungen der Formel Ib mit R gleich einer der Gruppen (a) bis (e) und $R_3$ gleich Wasserstoff bzw. $C_1$-$C_4$-Alkyl werden die entsprechenden Verbindungen der Formel Ia nach üblichen Methoden reduziert und, zur Herstellung der Verbindungen mit $R_3$ gleich Alkyl, sodann alkyliert.

10. Herstellung von Verbindungen der Formel Ia durch Ringschlußreaktion bei einer Verbindung der Formel

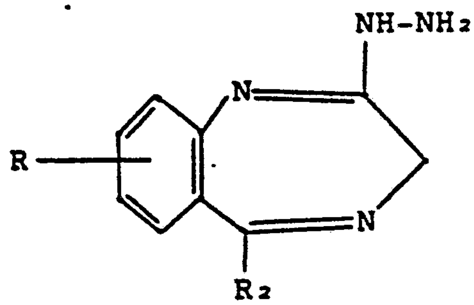

oder

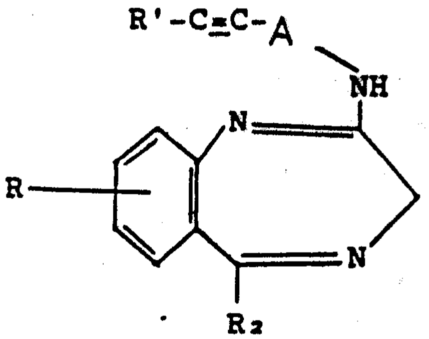

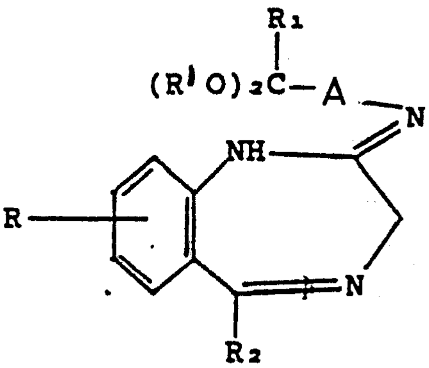

$A = CH_2$, $CHCH_3$

wobei R = -Z-COOR' oder -O-($C_1$-$C_4$)Alkyl

Verbindungen der allgemeinen Formel Ia, die einen [1,5-a] verknüpften Imidazolring (A Stickstoff und B = CH) enthalten, können beispielsweise in Analogieverfahren zu den in DE-OS 25 40 522 bzw. der niederländischen Patentanmeldung 78 03585 beschrieben, hergestellt werden.

11. Zur Herstellung von Verbindungen der Formel Ib, worin R den Rest der Formel IX bedeutet, setzt man funktionalisierte Verbindungen der Formel Ib, in denen R die Gruppe (d) bedeutet, mit geeigneten Reaktionspartnern um.

12. Verbindungen der allgemeinen Formel Ia mit R= O-X 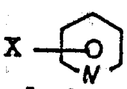 erhält man aus Verbindungen der allgemeinen Formel Ia mit R - OH durch Umsetzung mit einer Verbindung der Formel Halogen-X 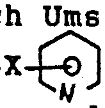 in Gegenwart einer Base. Die Deprotonierung der Hydroxyverbindungen mit der

Base, z.B. Natriumhydrid, Kaliumcarbonat oder ein Alkoholat erfolgt in einem inerten Lösungsmittel, wie z.B. Tetrahydrofuron, Methylethyleton, Aceton, Dimethylformamid oder geeigneten Kohlenwasserstoffen bei einer Temperatur zwischen -10°C und dem Siedepunkt des Reaktionsgemisches, bevorzugt bei Raumtemperatur, gegebenenfalls unter Schutzgas. Anschließend erfolgt die Zugabe der Halogenverbindung mit nachfolgender Aufarbeitung nach bekannten Methoden.

Soweit die erfindungsgemäßen Verbindungen ein asymmetrisch substituiertes Kohlenstoffatom aufweisen, können sie nach bekannten Methoden in ihre optisch aktiven Antipoden aufgetrennt werden, bei mehreren Asymmetriezentren in die Diastereomeren und deren Antipodenpaare.

Gewünschtenfalls werden aus den erhaltenen Verbindungen, soweit sie Basen sind, die Säureadditionssalze, soweit sie Säureadditionssalze sind, die Basen nach üblichen Verfahren hergestellt.

Die verwendeten Ausgangsstoffe der verschiedenen Verfahren sind bekannt oder nach üblichen Methoden in Analogieverfahren herzustellen.

Die erfindungsgemäßen Verbindungen besitzen PAF-antagonistische Wirkung.
Bekanntlich handelt es sich bei PAF (Plättchen Aktivierender Faktor) um das Phospholipid Acetyl-glyceryl-ether-phosphoryl-cholin (AGEPC), das als potenter Lipidmediator bekannt ist, der von tierischen und menschlichen proinflammatorischen Zellen freigesetzt wird. Unter solchen Zellen finden sich hauptsächlich basophile und neutrophile Granulozyten, Makrophagen (aus Blut und Gewebe) sowie Thrombozyten, die an Entzündungsreaktionen beteiligt sind.

PAF zeigt im pharmakologischen Experiment Bronchokonstriktion, Blutdrucksenkung, Auslösung einer Thrombozytenaggregation sowie eine proinflammatorische Wirkung.

Diese experimentell nachweisbaren Wirkungen des PAF weisen direkt oder indirekt auf mögliche Funktionen dieses Mediators in der Anaphylaxie, in der Pathophysiologie des Asthma bronchiale und allgemein in der Entzündung hin.

PAF-Antagonisten werden benötigt, um einerseits weitere pathophysiologische Funktionen dieses Mediators an Tier und Mensch aufzuklären und andererseits pathologische Zustände und Krankheiten, an denen PAF beteiligt ist, zu behandeln. Beispiele für die Indikationen eines PAF-Antagonisten sind Entzündungsprozesse des Tracheobronchialbaumes (akute und chronische Bronchitis, Asthma bronchiale) oder der Niere (Glomerulonephritis), der Gelenke (rheumatische Erkrankungen), anaphylaktische Zustände, Allergien und Entzündungen im Bereich der Schleimhäute und der Haut (z.B. Psoriasis) sowie durch Sepsis, Endotoxine oder Verbrennungen bedingte Schockzustände. Weitere wichtige Indikationen für einen PAF-Antagonisten sind Läsionen und Entzündungen im Bereich der Magen- und Darmschleimhaut, wie z.B. Gastritis, im allgemeinen Ulcus pepticum, jedoch insbesondere Ulcus ventriculi und Ulcus duodeni.

Weiterhin eignen sich die erfindungsgemäßen Verbindungen zur Behandlung bei den folgenden Indikationsstellungen:
Obstruktive Lungenerkrankungen, wie z.B. bronchiale Hyperreaktivität, entzündliche Lungenwegserkrankungen, wie z.B. chronische Bronchitis;
Herz- Kreislauferkrankungen, wie z.B. Polytrauma, Anaphylaxe, Arteriosklerose, entzündliche

Darmerkrankungen, EPH-Gestose (ederma-proteinuria Hypertension), Erkrankungen des extrakorporalen Kreislauf, ischämische Erkrankungen, entzündliche und immunologische Erkrankungen, Immunmodulation bei Transplantationen von Fremdgeweben, Immunmodulation bei Leukämie. Metastasenausbreitung z.B. bei bronchialer Neoplasie, Erkrankungen des ZNS, wie z.B. Migräne, Agarophobie (panic disorder), weiterhin erweisen sich die erfindungsgemäßen Verbindungen als Cyto- und Organoprotektiv, z.B. zur Neuroprotektion, z.B. bei Leberzirrhose, DIC (disiminierte intravasale Gerinnung);

Nebenwirkungen einer Arzneimitteltherapie, z.B. anaphylaktoide Kreislaufreaktionen, Kontrastmittelzwischenfälle, Nebenwirkungen bei der Tumortherapie;
Unverträglichkeiten bei Bluttransfusionen; fulminantes Leberversagen ($CCl_4$-Intoxikation)
Amanitaphalloides-Intoxikation (Knollenblätterpilzvergiftung);
Symptome von parasitären Erkrankungen (z.B. Wurmerkrankungen)

Neue zusätzliche Indikation: Immunfunktion bei Aids, Diabetes, juvenile Diabetes, diabetische Retinopathie, polytraumatischer Schock, hämorrhagischer Schock, CNS: Ishämie, Multiple Sklerose.

PAF assoziierte Interaktion mit Gewebshormon (autocoid hormones), Lymphokine und anderen Mediatoren.

Die PAF-antagonistische Wirkung einzelner Benzodiazepine ist bekannt, vgl. E. Kornecki et al, Science 226, 1454 - 1456 (1984). Für Alprazolam wurde nach der unten beschriebenen Methode ein $IK_{50}$ (Konzentration bei einer 50%igen Aggregationshemmung) von 14 µM, für Triazolam ein $IK_{50}$ von 9 µM gemessen. Diese, als Tranquilizer beziehungsweise Hypnotika ausgewiesenen und im Handel

befindlichen Verbindungen sind jedoch wegen ihrer ausgeprägten sedierenden Wirkung trotz ihrer relativ guten PAF-antagonistischen Wirkung zum Einsatz als PAF-Antagonisten in der Therapie in vielen Fällen ungeeignet.

Bei den erfindungsgemäßen Verbindungen hingegen fehlt die sedierenden Wirksamkeit, während die PAF-antagonistische Wirkung im Vergleich zu der der bekannten Benzodiazepine zumindest wesentlich besser ist.

Im folgenden werden die pharmakologischen Untersuchungsmethoden mitgeteilt:

Pharmakologische Untersuchungsmethoden

Die PAF-antagonistische Wirksamkeit einiger Verbindungen der Formel I wurde anhand der Hemmung der Blutplättchen-Aggregation in vitro und der Antagonisierung der durch PAF bewirkten Bronchokonstriktion am narkotisierten Meerschweinchen, Blutdrucksenkung an der narkotisierten Ratte und Hautquaddel an der Ratte untersucht. Darüber hinaus wurden diese Verbindungen hinsichtlich möglicher Nebenwirkungen auf das zentrale Nervensystem geprüft.

1. In vitro Untersuchungen: Hemmung der Blutplättchen-Aggregation

   Zur Bestimmung der PAF-antagonistischen Wirkung von Substanzen wurde die durch PAF induzierte Aggregation von Humanthrombozyten in vitro verwendet. Zur Gewinnung von thrombozytenreichem Plasma (TRP) erfolgt die Blutentnahme aus einer nicht gestauten Vene mit Hilfe einer Plastikspritze, in der sich 3,8 %ige Natriumcitratlösung befindet. Das Verhältnis zwischen Natriumcitratlösung und Blut beträgt 1:9. Nach vorsichtiger Durchmischung wird das Citratblut bei 150 x g (1200 U/min) 20 Minuten lang zentrifugiert. Die Messung der Thrombozytenaggregation erfolgt nach dem von Born und Cross ausgearbeiteten Verfahren (G. V. R. Born und M.J. Cross, J. Physiol. 168, 178 (1963)), wobei dem TRP unter ständigem Rühren PAF als Auslöser der Aggregation zugesetzt wird.

Die Prüfsubstanz wird jeweils 2 - 3 Minuten vor Auslösung der Aggregation in einem Volumen von 10 µl zugesetzt. Als Lösungsmittel dienen entweder Aqua.dest., Ethanol und/oder Dimethylsulfoxyd. Kontrollansätze erhalten entsprechende Volumina dieser Lösungsmittel. Nach Registrierung der Ausgangsabsorption (2-3 Minuten) wird die Aggregation mit PAF ($5 \times 10^{-8}$ M) induziert.

Zur Beurteilung von Substanzeffekten wird das Maximum der ersten Aggregationswelle verwendet. Die durch PAF induzierte maximale Absorptionsrate (= maximale Aggregation x 100 %) wird jeweils gleichzeitig in einem Parallelansatz (= Kontrollansatz in einem der Kanäle des 2 Kanal-Aggregometers) zu jedem Testansatz (zweiter Kanal) mitgeprüft und als 100 %-Wert verwendet.

Der unter dem Einfluß der Testsubstanz erreichte Aggregationswert wird als 100 % angegeben.

Jede Prüfsubstanz wird bei Konzentrationen von $10^{-3}$ bis $10^{-8}$ M mit einem Stichprobenumfang von jeweils n = 4 hinsichtlich einer hemmenden Wirkung auf die durch PAF induzierte Thrombozytenaggregation untersucht. Danach wird eine Konzentrations-Wirkungskurve anhand von 3 Konzentrationen erstellt und die $IK_{50}$ (Konzentration bei einer 50%igen Aggregationshemmung) ermittelt. Die IK-Werte von Verbindungen der allgemeinen Formel I bewegen sich im allgemeinen um Werte, die kleiner als 9 µM sind.

## 2. In vivo Untersuchungen

### 2.1. Antagonisierung der durch PAF bewirkten Bronchokonstriktion an narkotisierten Meerschweinchen

Spontan atmende männliche, 300 - 450 g schwere Meerschweinchen werden 1 Stunde vor einer i.v. Infusion von PAF (30 ng/(kg x min) mit der Testsubstanz oder einem Kontrollvehikel oral vorbehandelt. Die Versuchstiere werden dann mit 2 mg/kg Urethan intraperitoneal anästhesiert, worauf die Vena jugularis, die Arteria carotis und die Trachea kanüliert werden. Die PAF-Infusion induziert bei den Kontrolltieren eine starke, anhaltende Bronchokonstriktion, die anhand des Atemzugvolumens, der Compliance und der Resistance gemessen wird und ebenso eine Senkung des Blutdruckes. Nach ca. 7 - 10 Minuten tritt der Tod ein. Mit den beschriebenen PAF-Antagonisten können diese Effekte auf Atmung und Blutdruck sowie der Eintritt des Todes verhindert werden.

### 2.2. Antagonisierung der durch PAF bewirkten Blutdrucksenkung an der narkotisierten Ratte

Normotone, männliche, 200 - 250 g schwere Wistar-Ratten werden mit 2 mg/kg Urethan intraperitoneal anästhesiert. Die Arteria carotis und Vena jugularis werden kanüliert. Eine intravenöse PAF-Infusion (30 ng/(kg x min)) induziert bei den Kontrolltieren eine starke, anhaltende Blutdrucksenkung. Diese kann dosisabhängig durch intravenöse Injektionen (kumulative Verabreichung) der beschriebenen

**Verbindungen aufgehoben werden. Auch eine orale oder intravenöse Verabreichung der Verbindung vor Beginn der PAF-Infusion kann die blutdrucksenkende Wirkung der oben genannten PAF-Infusion dosisabhängig verhindern.**

2.3. Antagonisierung der durch PAF induzierten Hautquaddel an der Ratte (Modifiziert nach P.P. Koelzer und K.H. Wehr, Arzneim.-Forsch. 8, 181 (1958)

Eine intrakutane Injektion von PAF induziert eine Hautquaddel, die Ausdruck einer durch PAF bedingten Erhöhung der Gefäßpermeabilität ist.

Männlichen Wistar-Ratten mit einem Körpergewicht von 250 ± 20 g wird die Bauchdecke (kurz) geschoren. Danach werden 1 ml/kg einer 1%igen Trypanblau-Lösung durch eine Schwanzvene den Tieren injiziert. Symmetrisch zur Mittellinie (linea alba) werden an drei Stellen in Abständen von ca. 1,5 cm intrakutane Injektionen von physiologischer Kochsalzlösung oder PAF-Lösung (12,5 bis 15,0 ng/Stelle in 0,1 ml) verabreicht. Während an der Injektionsstelle der Kochsalzlösung keine Reaktion auftrat, bewirkte PAF eine Hautreaktion (Quaddel), die durch eine Blaufärbung unterschiedlicher Intensität - abhängig von der PAF-Dosis - sichtbar wurde. Durch gleichzeitige intrakutane Verabreichung der beschriebenen Verbindungen oder durch eine intravenöse Vorbehandlung konnte diese durch PAF induzierte Hautreaktion verhindert werden..

3. Wirkungen auf das zentrale Nervensystem

Es ist allgemein bekannt, daß Substanzen dieses Strukturtyps zentralnervöse Effekte verursachen, die jedoch für eine Verbindung mit PAF-antagonistischer Wirkung nicht erwünscht sind. Daher wurden die beschriebenen Verbindungen hinsichtlich ihrer hypnogenen und antikonvulsiven Wirkung sowie ihres Einflusses auf die Lokomotion geprüft. Eine mögliche hypnotische Wirkung wurde an 400 bis 450 g schweren Meerschweinchen untersucht. Dosen bis zu 200 mg/kg p.o. dieser Substanzen waren nicht in der Lage, eine hypnotische oder sedative Wirkung an diesen Tieren zu erzeugen.
Zur Prüfung einer antikonvulsiven Wirkung kann der Pentetrazol-Antagonismus bei Mäusen (20 bis 25 g Körpergewicht) verwendet werden (M. I. Gluckmann, Current Therapeutic Research, 7:721, 1965). Dosen bis zu 100 mg/kg p.o. dieser Verbindungen (1 Stunde vor Pentetrazol) zeigten in diesem Test keinen Einfluß auf die durch Pentetrazol (125 mg/kg i.p., LD 100) hervorgerufene Mortalität.
Die Wirkung auf die Nachtmotilität (Lokomotion) von Mäusen (20 - 25 g Körpergewicht) kann in einem Lichtschrankenkäfig untersucht werden. Dabei wird die Anzahl der Lichtstrahlunterbrechungen gemessen. Dosen bis zu 300 mg/kg p.o. der oben genannten Verbindungen zeigten keine Aktivität.

In der nachstehenden Tabelle sind die PAF-Werte und Werte für den Flunitrazepam-Bindungstest einiger erfindungsgemäßer und einiger bekannter Verbindungen angegeben.

Tabelle A

| Verbindung | $IK_{50}$-Wert PAF $10^{-6}$[mol] | $IK_{50}$-Wert FNB $10^{-9}$[mol] |
|---|---|---|
| (1) Stand der Technik: | | |
| Alprazolam | 14 | 5.5 |
| Triazolam | 9 | 1.4 |
| Verbindung Nr.43 Seite 17 des südafrikanischen Patents 85/7523 | 33,3 | 10,7 |
| (2) Erfindungsgemäß: | | |
| (a) | 1,3 | 570 |
| (b) | 0,4 | 40 |
| (c) | 0,8 | 2160 |
| (d) | 0,3 | 2680 |
| (e) | 0,3 | - |
| (f) | 0,4 | 800 |
| (g) | 0,9 | > 5000 |
| (h) | 1,2 | - |
| (i) | 0,4 | 4550 |
| (j) | 0.1 | >5000 |
| (k) | 0.2 | 1420 |
| (l) | 0.1 | >5000 |
| (m) | 0.1 | - |

Bei (a) bis (m) handelt es sich um folgende Verbindungen:

<u>Gemäß Formel Ia</u>
(mit $R_1$ gleich $CH_3$, $R_2$ gleich 2-Chlorphenyl)

| Verbindung | A | B | R (Pos. 8) |
|---|---|---|---|
| (a) | N | N | $-O-(CH_2)_3-COOC_2H_5$ |
| (b) | N | N | $-O-CH_2-CO-NH-i-C_4H_9$ |
| (c) | N | N | $-O-CH_2-CO-NH-$ 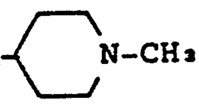 |
| (d) | N | N | $-O-CH_2-CH_2-$ 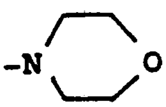 |
| (e) | N | N | $-O-CH_2-CH_2-$ 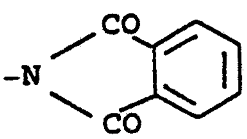 |
| (f) | N | N | 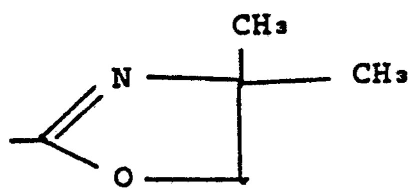 |
| (g) | N | N | $-CO-NH-(CH_2)_2-$ 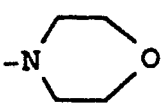 |
| (h) | N | N | $-CO-O(CH_2)_3N(CH_3)_2$ |

| Verbindung | A | B | R | |
|---|---|---|---|---|
| (j) | N | N | -O-(CH)₃- 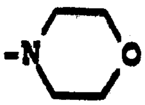 | $R_3$ = $C_6H_5$ |
| (k) | N | N | -O-$CH_2$- 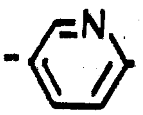 | $R_3$ = $C_6H_5$ |
| (l) | CH | N | -O-(CH)₃- 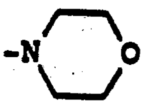 | $R_3$ = $C_6H_5$ |
| (m) | CH | N | -O-$CH_2$- 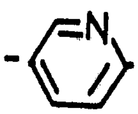 | $R_3$ = $C_6H_5$ |

Gemäß Formel Ib

| Verbindung | A | B | R | $R_1$ | $R_2$ | $R_3$ |
|---|---|---|---|---|---|---|
| (i) | N | N | -O-$CH_2$-$CH_2$- 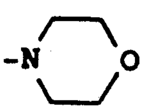 | $CH_3$ | 2-Cl-$C_6H_4$ | H |

Die neuen Verbindungen der allgemeinen Formeln Ia oder Ib können topisch, oral, transdermal, parenteral oder durch Inhalation verabreicht werden. Die Verbindungen liegen hierbei als aktive Bestandteile in gebräuchlichen Darreichungsformen vor, z.B. in Zusammensetzungen, die im wesentlichen aus üblichen pharmazeutischen Träger- und/oder Hilfsstoffen und einer effektiven Dosis des Wirkstoffes bestehen, wie z.B. Tabletten, Dragees, Kapseln, Oblaten, Pulver, Lösungen, Suspensionen, Inhalationsaerosole, Salben, Emulsionen, Sirupe, Suppositorien. Eine wirksame Dosis der erfindungsgemäßen Verbindungen liegt bei oraler Anwendung zwischen 1 und 50, vorzugsweise zwischen 3 und 20 mg/Dosis, bei intravenöser oder intramuskulärer Anwendung zwischen 0,01 und 50, vorzugsweise zwischen 0,1 und 10 mg/Dosis. Für die Inhalation sollen Lösungen, die 0,01 bis 1,0, vorzugsweise 0,1 bis 0,5 % Wirkstoff enthalten, eingesetzt werden.

Nachfolgend werden Beispiele für einige pharmazeutische Zusammensetzungen unter Verwendung von Verbindungen der allgemeinen Formel Ia oder Ib als aktiver Bestandteil angegeben. Falls nicht ausdrücklich anders bezeichnet, handelt es sich bei den Teilen um Gewichtsteile.

1. Tabletten

Die Tablette enthält folgende Bestandteile:

| | |
|---|---|
| Wirkstoff gemäß Formel Ia/Ib | 0,020 Teile |
| Stearinsäure | 0,010 " |
| Dextrose | 1,890 " |
| gesamt | 1,920 Teile |

Herstellung:

Die Stoffe werden in bekannter Weise zusammengemischt und die Mischung zu Tabletten verpreßt, von denen jede 1,92 g wiegt und 20 mg Wirkstoff enthält.

2. Salbe

Die Salbe setzt sich aus folgenden Bestandteilen zusammen:

| | |
|---|---|
| Wirkstoff gemäß Formel Ia/Ib | 50 mg |
| Neribas Salbe (Handelsware Scherax) | ad 10 g |

Herstellung:

Der Wirkstoff wird mit 0,5 g Salbengrundlage verrieben und die restliche Grundlage in Teilmengen zu 1,0 g nach und nach innig zu einer Salbe vermischt. Man erhält eine 0,5 %ige Salbe. Die Verteilung des Wirkstoffes in der Grundlage wird optisch unter dem Mikroskop kontrolliert.

3. Creme

Zusammensetzung:

| | | |
|---|---|---|
| Wirkstoff gemäß Formel Ia/Ib | | 50 mg |
| Neribas Salbe (Handelsware Scherax) | ad | 10 g |

Herstellung

Der Wirkstoff wird mit 0,5 g Cremegrundlage verrieben und die restliche Grundlage in Teilmengen zu 1,0 g nach und nach mit Pistill eingearbeitet. Man erhält eine 0,5%ige Creme. Die Verteilung des Wirkstoffes in der Grundlage wird optisch unter dem Mikroskop kontrolliert.

4. Ampullenlösung

Zusammensetzung:

| | | |
|---|---|---|
| Wirkstoff gemäß Formel Ia/Ib | | 1,0 mg |
| Natriumchlorid | | 45,0 mg |
| Aqua pro inj. | ad | 5,0 ml |

Herstellung:

Der Wirkstoff wird bei Eigen-pH oder gegebenenfalls bei pH 5,5 bis 6,5 in Wasser gelöst und Natriumchlorid als Isotonanz zugegeben. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 1 mg, 5 mg und 10 mg Wirkstoff.

5. Suppositorien

Jedes Zäpfchen enthält:

| | |
|---|---|
| Wirkstoff gemäß Formel Ia/Ib | 1,0 Teile |
| Kakaobutter (Fp.36-37°C) | 1200,0 Teile |
| Carnaubawachs | 5,0 Teile |

Herstellung

Kakaobutter und Carnaubawachs werden zusammengeschmolzen. Bei 45°C gibt man den Wirkstoff hinzu und rührt, bis eine komplette Dispersion entstanden ist.
Die Mischung wird in Formen entsprechender Größe gegossen und die Zäpfchen zweckmäßig verpackt.

6. Inhalationslösungen

Zusammensetzung:

| | | |
|---|---|---|
| a)Wirkstoff gemäß Formel Ia/Ib | | 500 mg |
| Na-EDTA | | 50 mg |
| Benzalkoniumchlorid | | 25 mg |
| Natriumchlorid | | 880 mg |
| destilliertes Wasser | ad | 100 ml |

Herstellung:

96 % der Wassermenge werden vorgelegt, darin nacheinander Na-EDTA, Benzalkoniumchlorid, Natriumchlorid und Wirkstoff klar gelöst und mit dem restlichen Wasser aufgefüllt. Die Lösung wird in 20 ml-Tropfflaschen abgefüllt. Eine Dosis (20 Tropfen, 1 ml) enthält 5 mg Wirkstoff.

| | | |
|---|---|---|
| b) Wirkstoff gemäß Formel Ia/Ib | | 500 mg |
| Natriumchlorid | | 820 mg |
| destilliertes Wasser | ad | 100 ml |

Herstellung

96 % der Wassermenge werden vorgelegt, darin nacheinander der Wirkstoff und Natriumchlorid gelöst, mit dem restlichen Wasser aufgefüllt und die Lösung in Eindosenbehälter (4 ml) abgefüllt. Die Lösung enthält 20 mg Wirkstoff.

**Beispiel 1**

**6-(2-Chlorphenyl)-8-(2-imidazolin-2-yl)-1-methyl-4H-[1,2,4]triazolo[4,3-a][1,4]benzodiazepin**

3,5 g (11 mmol) 8-Cyano-6-(2-chlorphenyl)-1-methyl-4H-[1,2,4]triazolo[4,3-a][1,4]benzodiazepin werden mit 7 g einer 30%igen ethanolischen Salzsäure versetzt und unter Feuchtigkeitsausschluß 6 Tage im Kühlschrank aufbewahrt, wobei gelegentlich die Suspension umgeschüttelt wird. Man engt im Vakuum ein und verarbeitet das rohe Imidoesterhydrochlorid direkt weiter. Dazu gibt man auf den Rückstand 6 g (0,1 mol) Ethylendiamin und erhitzt 3 Stunden bei 80°C. Man verdünnt nach dem Abkühlen mit Dichlormethan, wäscht mehrfach mit Wasser, trocknet die organische Phase und engt ein.
Der Rückstand wird aus Ether umkristallisiert
Ausbeute 1 g vom Fp. 186-189°C.

Die Ausgangsverbindung erhält man auf folgendem Wege.

Ausgehend von 2-Acetamido-5-methyl-2'-chlorbenzophenon erhält man, analog der Vorschrift für 1,3-Dihydro-5-phenyl-7-methoxycarbonyl-2H-[1,4]benzodiazepin-2-on (Sternbach et.al.Helv. Chim. Acta 186, 1720 (1969)), 1,3-Dihydro-5-(2-chlorphenyl)-7-methoxycarbonyl-2H-[1,4]benzodiazepin-2-on vom Fp. 245-248°C.

37,5 g (0,114 mol) dieses Benzodiazepinons werden in 350 ml Pyridin mit 32 g Phosphorpentasulfid 5 Stunden auf 65 °C erhitzt. Die Reaktionsmischung wird sodann in 700 ml einer 20%igen Kochsalzlösung eingerührt, dann mit Wasser verdünnt und das ausgefallene Benzodiazepin-2-thion abfiltriert und mit Ethanol gewaschen. Das Rohprodukt, 39 g, kann ohne weitere Reinigung weiter umgesetzt werden.

Umkristallisieren aus Isopropylether führt zum Thion vom Fp. 246-248°C.

39 g (0,113 mol) des Thions werden in 500 ml Tetrahydrofuran mit 10 g Hydrazinhydrat versetzt und 30 Minuten bei Raumtemperatur gerührt. Die Suspension wird über Kieselgur filtriert und zur Trockne eingeengt. Das Rohprodukt wird in 540 ml wasserfreiem Ethanol gelöst; nach Zugabe von 106 ml Orthoessigsäuretriethylester erhitzt man 1 Stunde auf Rückflußtemperatur. Nach Eindampfen des Lösungsmittels und Umkristallisieren aus Essigester erhält man 33 g des 6-(2-Chlorphenyl)-1-methyl-8-(methoxycarbonyl)-4H-[1,2,4]triazolo[4,3-a]-[1,4]benzodiazepins vom Fp. 169-173°C.

Eine analysenreine Substanz erhält man durch Umkristallisieren aus Methanol/Ether: Fp. 178-180°C.

Die Verbindung kann auch aus dem Benzodiazepin-2-thion mit Essigsäurehydrazid hergestellt werden.

15 g (40 mmol) 6-(2-Chlorphenyl)-8-methoxycarbonyl-1-methyl-[1,2,4]triazolo[4,3-a][1,4]benzodiazepin werden in 750 ml gesättigter methanolischer Ammoniaklösung 2 Tage gerührt. Man filtriert das ausgefallene Carbonsäureamid ab: 9,7 g (69 %) vom Fp. 308-310°C.

9,7 g (28 mmol) des 8-Aminocarbonyl-6-(2-chlorphenyl)-1-methyl-[1,2,4]triazolo[4,3-a][1,4]benzodiazepins erhitzt man 5 Stunden unter Rückfluß in einer Mischung aus 150 ml Dichlorethan und 10 m. Phosphoroxychlorid. Man gießt anschließend auf Eis, trennt die organische Phase ab und wäscht diese nochmals mit Wasser. Nach üblicher Aufarbeitung der organischen Phase erhält man durch Umkristallisieren aus Essigester 7 g (75 %) des Cyanids vom Fp. 240-242°C.

Beispiel 2

6-(2-Chlorphenyl)-8-(4,4-dimethyloxazolin-2-yl)-1-methyl-4H-[1,2,4]triazlolo[4,3-a][1,4]benzodiazepin

33 g (0,09 mol) des in Beispiel 1 beschriebenen 6-(2-Chlor-phenyl)-8-methoxycarbonyl-1-methyl-4H-[1,2,4]-triazolo[4,3-a][1,4]benzodiazepins werden mit 4,5 g (0,113 mol) Natriumhydroxid in einer Mischung aus 120 ml Wasser, 400 ml Tetrahydrofuran und 400 ml Methanol bei Siedetemperatur in 1 Stunde verseift. Man engt die Mischung ein, nimmt mit Wasser auf und säuert mit Eisessig an. Die ausgefallenen Kristalle werden isoliert und getrocknet.

Ausbeute 28,6 g (90 %) 8-Carboxy-6-(2-chlorphenyl)-1-methyl-[1,2,4]triazolo[4,3-a][1,4]benzodiazepin vom Fp. 350-352°C.

1,75 g (5 mmol) der so gewonnenen Benzodiazepincarbonsäure werden zusammen mit 0,45 g (mmol) 2-Amino-2-methyl-1-propanol und 1,5 g (mmol) Triethylamin in einer 10 ml Mischung aus wasserfreiem Pyridin/Acetonitril (1:1) vorgelegt. Bei Raumtemperatur tropft man dazu 4 g Triphenylphosphin gelöst in 10 ml der gleichen Lösungsmittelmischung innerhalb von 3 h zu. Nach 24 h wird die Suspension eingeengt und der Rückstand mit Ether, dann mit Essigester extrahiert, die Extrakte werden vereinigt und nach dem Abdestillieren der Lösungsmittel an Kieselgel chromatographiert (Eluens: Dichlormethan/Methanol 95/5). Die sauberen Fraktionen werden nach dem Eindampfen aus Ether kristallisiert. Ausbeute: 1,5 g (75 %) vom Fp. 235-236°C.

Die Titelverbindung ist auch auf folgendem Wege zugänglich:

1 g (2,3 mmol) 6-(2-Chlorphenyl)-8-[(1,1-dimethyl-2-hydroxyethyl)]aminocarbonyl]-1-methyl-4H-[1,2,4]triazolo-[4,3-a][1,4]benzodiazepin werden in 50 ml Dichlormethan mit 0,6 ml Thionylchlorid versetzt und 5 h bei Raumtemperatur gerührt. Nach Einengen wird mit Eis versetzt und alkalisch gestellt. Man extrahiert die Mischung mehrfach mit Dichlormethan und arbeitet die organische Phase wie üblich auf. Man kristallisiert den Rückstand aus Ether und erhält 0,7 g (75 %) des Dimethyloxazolins.

Das obige Ausgangsamid erhält man durch Umsetzung der im Beispiel 1 beschriebenen Carbonsäure mit 2-Amino-2-methyl-1-propanol nach dem bekannten Carbonyldiimidazolverfahren. Fp. 172 - 175°C (Hydrat)

Beispiel 3

6-(2-Chlorphenyl)-8-(4,4-dimethylthiazolin-2-yl)-1-methyl-4H-[1,2,4]triazolo[4,3-a][1,4]benzodiazepin

1 g (2,5 mmol) der in Beispiel 2 gewonnenen Dimethyloxazolinverbindung erhitzt man in 5 ml Xylol zusammen mit 2 g (9 mmol) Phosphorpentasulfid. Nach dem Abkühlen verdünnt man mit Dichlormethan und schüttelt mit Wasser aus. Die organische Phase wird wie üblich aufgearbeitet. Den Rückstand reinigt man chromatographisch an Kieselgel mit Eluens Dichlormethan/Methanol (95:5). Die sauberen Fraktionen werden aus Essigester/Ether umkristallisiert.

Ausbeute 0,6 g (57 %) des Thiazolins vom Fp. 235°C.

## Beispiel 4

8-Methoxycarbonyl-6-(2-chlorphenyl)-1-methyl-4H-imidazo-[1,2-a][1,4]benzodiazepin

Zu 3,1 g (9 mmol) 5-(2-Chlorphenyl)-7-methoxycarbonyl-1H,3H-[1,4]benzodiazepin-2-thion in 60 ml wasserfreiem Dioxan tropft man 1,6 g Propargylamin und erhitzt anschließend 3 Stunden unter Rückfluß und rührt eine weitere Stunde bei Raumtemperatur. Der nach Abziehen des Lösungsmittels verbliebene Rückstand wird in Dichlormethan/Wasser aufgenommen. Die wäßrige Phase wird nochmals mit Dichlormethan extrahiert, die gesammelten organischen Phasen mit Wasser gewaschen, das Lösungsmittel entfernt und der Rückstand aus Essigester/Ether umkristallisiert. Man erhält 2,1 g 5-(2-Chlorphenyl)-7-(methoxycarbonyl)-3H-2-propargylamino-[1,4]benzodiazepin vom Fp. 202-205°C.

1,6 g (4,37 mmol) der so erhaltenen Verbindung werden in 7,5 ml konzentrierter Schwefelsäure 10 Minuten auf 100°C erhitzt. Nach dem Abkühlen gießt man auf Eis, alkalisiert mit Ammoniaklösung und extrahiert mit Dichlormethan.

Die wäßrige Phase wird auf pH=5 eingestellt und das ausgefallene 8-Carboxy-6-(2-chlorphenyl)-1-methyl-4H-imidazo[1,2-a][1,4]benzodiazepin abfiltriert und getrocknet.

Ausbeute: 1 g (65 % d.Th.); Fp. 295-297°C.

Die organische Phase wird eingeengt. Man erhält 0,2 g (13 % d.Th.) 8-Methoxycarbonyl-6-(2-chlorphenyl)-1-methyl-4H-imidazo[1,2-a][1,4]benzodiazepin vom Fp. 150-151°C.

Beispiel 5

a) 8-(3-Ethoxycarbonyl-propyloxy)-1-methyl-6-(2-chlorphenyl)-4H-[1,2,4]triazolo[4,3-a][1,4]benzodiazepin

Eine Suspension von 2 g (0,006 Mol) 8-Hydroxy-1-methyl-6-(2-chlorphenyl)-4H-[1,2,4]triazolo-[4,3-a][1,4]benzodiazepin und 2,48 g wasserfreies Kaliumcarbonat in 55 ml Ethylmethylketon wird eine Stunde unter Rühren zum Rückfluß erhitzt. Man läßt die Reaktionsmischung abkühlen, tropft eine Lösung von 1,29 g (0,0066 Mol) 4-Brombuttersäureethylester zu und erhitzt weitere sieben Stunden zum Sieden. Das Lösungsmittel wird im Vakuum abdestilliert und der verbleibende Rückstand zwischen Wasser und Methylenchlorid verteilt. Die organische Phase wird nacheinander mit kalter 5%iger Natronlauge und Wasser ausgeschüttelt, über Natriumsulfat getrocknet und im Vakuum eingeengt. Man erhält 1,6 g (61 % d.Th.) der Titelverbindung vom Schmelzpunkt 157 - 159°C (Essigester).

$C_{23}H_{23}ClN_4O_3$ (438,93)

| | | | | |
|---|---|---|---|---|
| Ber.: | C 62.94 | H 5.28 | Cl 8.08 | N 12.76 |
| Gef.: | 62.73 | 5.29 | 7.94 | 12.68 |

Analog werden erhalten:

b) 8-(Ethoxycarbonyl-methoxy)-1-methyl-6-(2-chlorphenyl)-4H-[1,2,4]triazolo[4,3-a][1,4]benzodiazepin

Schmelzpunkt: 167 - 170°C.

$C_{21}H_{19}ClN_4O_3$ (410.87)

| | | | |
|---|---|---|---|
| Ber.: | C 61.39 | H 4.66 | N 13.64 |
| Gef.: | 60.97 | 4.61 | 13.48 |

c) 8-(Ethoxycarbonyl-methoxy)-1-methyl-6-phenyl-4H-[1,2,4]-triazolo[4,3-a][1,4]benzodiazepin

Schmelzpunkt: 207 - 208°C (Acetonitril).

$C_{21}H_{20}N_4O_3$ (376.42)

| | | | |
|---|---|---|---|
| Ber.: | C 67.01 | H 5.36 | N 14.88 |
| Gef.: | 66.91 | 5.34 | 14.91 |

d) 8-(1-Ethoxycarbonyl-ethoxy)-1-methyl-6-(2-chlorphenyl)-4H-[1,2,4]triazolo[4,3-a][1,4]benzodiazepin

$^1$H-NMR ($CDCl_3$ TMS int.): δ 1,18 1,25 (t 3H, 7Hz, $CH_3$, 1.59 (d, 3H, 7Hz, $CH_3$) 2.59 (s, 3H, $CH_3$), 3.90 -4.40 (m, 2H, $-CH_2-O-$), 4.18 (d, 1H, $-CH_2-$, A/B System $J_{AB}$ 12Hz), 4.66 (m, 1H, -CH<), 5.52 (d, 1H, $-CH_2-$, A/B System $J_{AB}$ 12Hz), 6.68 (m, 1H, arom.), 7.02- 7.71 (m, 6H, arom.)

e) 8-(1-Ethoxycarbonyl-ethoxy)-1-methyl-6-phenyl-4H-[1,2,4]triazolo[4,3a][1,4]benzodiazepin

Schmelzpunkt: 184 - 186° (Essigester/Benzin)
$C_{22}H_{22}N_4O_3$ (390,45)

| | | | |
|---|---|---|---|
| Ber.: | C 67.68 | H 5.68 | N 14.35 |
| Gef.: | 67.35 | 5.60 | 14.27 |

f) 8-(Cyano-methoxy)-1-methyl-6-phenyl-4H-[1,2,4]triazolo-[4,3-a][1,4]benzodiazepin

Fp.: 185 - 186° (Acetonitril/Diisopropylether)
$C_{19}H_{15}N_5O$ (329.37)

| | | | |
|---|---|---|---|
| Ber.: | C 69.29 | H 4.59 | N 21.26 |
| Gef.: | 69.27 | 4.62 | 21.40 |

g) 8-(Ethoxycarbonyl-methoxy)-1-methyl-6-phenyl-4H-imidazo-[1,2-a][1,4)benzodiazepin
durch Umsetzung von 8-Hydroxy-1-methyl-6-phenyl-4H-imidazo[1,2-a][1,4]benzodiazepin mit Bromessigsäureethylester

Fp. 182 - 184° (Acetonitril)
$C_{22}H_{21}N_3O_3$ (375.43)

| | | | |
|---|---|---|---|
| Ber.: | C 70.38 | H 5.64 | N 11.19 |
| Gef.: | 70.15 | 5.59 | 11.23 |

Beispiel 6

a) [1-Methyl-6-phenyl-4H-[1,2,4]triazolo[4,3a][1,4]benzodiazepin-8-yl]oxyessigsäure

6 g (0,016 Mol) 8-(Ethoxycarbonyl-methoxy)-1-methyl-6-phenyl-4H-[1,2,4]triazolo[4,3-a][1,4]benzodiazepin werden mit 0,8 g (0.02 Mol) Natriumhydroxid in einer Mischung von 50 ml Alkohol und 120 ml Wasser bei Raumtemperatur in einer Stunde verseift. Man engt die Reaktionslösung ein, nimmt den Rückstand mit Wasser auf und stellt die Lösung unter Eiskühlung mit 2 n Salzsäure schwach sauer. Die anfallende Carbonsäure wird isoliert und getrocknet.

Ausbeute: 4,6 g (83 % d.Th.); Fp. 260 - 263°C.

Analog werden hergestellt:

b) [1-Methyl-6-(2-chlorphenyl)-4H-[1,2,4]triazolo[4,3-a]-[1,4]benzodiazepin-8-yl]oxyessigsäure

Schmelzpunkt: 236° - 238°C.

c) 2-{[1-Methyl-6-(2-chlorphenyl)-4H-[1,2,4]triazolo-[4,3-a][1,4]benzodiazepin-8-yl]oxy}propionsäure

$^1$H-NMR ($CD_4O$, TMS int.): δ 1.52 (d, d, 3H, $CH_3$, J 7Hz), 2.56 (s, 3H, $CH_3$), 4.31 (d, 1H, $-CH_2-$, A/B System $J_{AB}$ 12 Hz), 4.72 (q, 1H, -CH<), 5.27 (d, 1H, $-CH_2-$, A/B System $J_{AB}$ 12Hz), 6.68 (d, 1H, arom. J 3Hz), 7.18-7.80 (m, 6H, arom.)

d) [1-Methyl-6-phenyl-4H-imidazo[1,2a][1,4]benzodiazepin-8-yl]oxyessigsäure
aus
8-(Ethoxycarbonyl-methoxy)-1-methyl-6-phenyl-4H-imidazo-[1,2-a][1,4]benzodiazepin
Schmelzpunkt: 270° Z.

**Beispiel 7**

a) 1-Methyl-8-(2-morpholin-4-yl-ethoxy)-6-(2-chlorphenyl)-4H-[1,2,4]triazolo[4,3-a][1,4]benzodiazepin

Ein Gemisch von 3,25 g (0,01 Mol) 8-Hydroxy-1-methyl-6-(2-chorphenyl)-4H-[1,2,4]triazolo-[4,3-a][1,4]benzodiazepin, 5,5 g wasserfreiem Kaliumcarbonat und 100 ml Ethylmethylketon wird unter Rühren eine Stunde zum Sieden erhitzt. Nach dem Abkühlen wird die Reaktionsmischung mit 2,1 g (0,011 Mol) N-(2-Chloräthyl)-morpholin-hydrochlorid versetzt und 22 Stunden unter Rückfluß erhitzt. Das Solvens wird abgezogen, der Rückstand mit Methylenchlorid/Wasser aufgenommen und die organische Phase nacheinander mit 5%iger Natronlauge und Wasser gewaschen. Die Methylenchlorid-Lösung wird getrocknet und unter vermindertem Druck eingedampft. Der ölige Rückstand wird säulenchromatographisch über Kieselgel gereinigt, wobei zunächst Aceton und dann ein Gemisch von Aceton und Ethanol (80 : 20) als Eluens benutzt wird. Beim Einengen der Hauptfraktionen erhält man ein zähes Öl, das mit Diisopropyläther kristallisiert. Nach dem Umkristallisieren aus Essigester erhält man 2,6 g (60 % d.Th.) der gewünschten Verbindung vom Schmelzpunkt 145 - 146°C.

$C_{23}H_{24}ClN_5O_2$ (437,94)

| | | | | |
|---|---|---|---|---|
| Ber.: | C 63.08 | H 5.52 | Cl 8.10 | N 15.99 |
| Gef.: | 62.84 | 5.49 | 7.81 | 15.91 |

Analog werden erhalten:

b) 1-Methyl-8-(3-morpholin-4-yl-propyloxy)-6-(2-chlorphenyl)-4H-[1,2,4]triazolo[4,3-a][1,4]benzodiazepin

$^1$H-NMR ($CDCl_3$, TMS int.): δ 1.72 - 2.12 (m, 2H, $-CH_2-$), 2.43 (m, 6H, $-CH_2-N<$, $2-CH_2$-Morpholin), 2.61 (s, 3H, $CH_3$). 3.70 (m, 4H, $-CH_2$-Morpholin), 4.00 (t, 2H, J 6Hz, $-CH_2-O-$), 4.18 (d, 1H, $-CH_2-$ A/B System $J_{AB}$ 12 Hz), 5.52 (d, 1H, $-CH_2-$, A/B System $J_{AB}$ 12Hz), 6.70 (d 1H arom., J 3Hz), 7.06 - 7.72 (m, 6H arom.)

c) 1-Methyl-8-(2-morpholino-ethoxy)-6-phenyl-4H-[1,2,4]-triazolo[4,3-a][1,4]benzodiazepin

Schmelzpunkt: 141 - 143° (Essigester/Äther)

$C_{23}H_{25}N_5O_2$ (403.49)

| | | | |
|---|---|---|---|
| Ber.: | C 68.47 | H 6.25 | N 17.36 |
| Gef.: | 68.59 | 6.35 | 17.29 |

d) 8-(2-Dimethylamino-ethoxy)-1-methyl-6-(2-chlorphenyl)-4H-[1,2,4]triazolo[4,3-a][1,4]benzodiazepin

$^1$H-NMR ($CDCl_3$, TMS int.): δ 2.32 (s, 6H, $N-CH_3$); 2.63 (s, 3H, $CH_3$), 2.70 (t, 2H, $-CH_2-$, J 6Hz), 4.00 (t, 2H, $-CH_2-$. J 6Hz), 4.18 (d, 1H, $-CH_2-$, A/B System $J_{AB}$ 12 Hz), 5.50 (d, 1H, -CH-, A/B System, $J_{AB}$ 12Hz), 6.75 (d 1, H arom. J 3Hz), 7.09 - 7.73 (m, 6H, arom.)

Beispiel 8

a) 8-(Ethoxycarbonyl-methoxy)-1-methyl-6-(2-chlorphenyl)-5,6-dihydro-4H-[1,2,4]triazolo[4,3-a][1,4]benzodiazepin

Man versetzt eine Lösung von 1 g (0.0024 Mol) 8-(Ethoxycarbonyl-methoxy)-1-methyl-6-(2-chlorphenyl)-4H [1,2,4]triazolo[4,3-a][1,4]benzodiazepin in einer Mischung von je 15 ml Dichlormethan und Eisessig unter Rühren mit 0,8 g Zinkstaub und läßt sechs Stunden bei Raumtemperatur reagieren. Die Suspension wird über Kieselgel abfiltriert und mit Dichlormethan nachgewaschen. Die vereinigten Filtrate werden mit verdünnter Ammoniaklösung unter Kühlung alkalisch gestellt. Die organische Phase wird mit Wasser gewaschen, getrocknet und eingedampft. Das zurückbleibende dunkle Öl wird durch chromatographische Aufarbeitung an Kieselgel (Aceton) gereinigt.

Ausbeute: 300 mg (30 % d.Th.); Fp. 187 - 189°C.

$C_{21}H_{21}ClN_4O_3$ (412.88)

| | | | | |
|---|---|---|---|---|
| Ber.: | C 61.09 | H 5.13 | Cl 8.59 | N 13.57 |
| Gef.: | 60.74 | 5.17 | 8.77 | 13.29 |

Beispiel 9

1-Methyl-8-(2-morpholin-4-yl-ethoxy)-6-(2-chlorphenyl)-5,6-dihydro-4H-[1,2,4]triazolo[4,3-a][1,4]benzodiazepin

1 g (0,016 Mol) 1-Methyl-8-(2-morpholin-4-yl-ethoxy)-6-(2-chlorphenyl)-4H-[1,2,4]triazolo[4,3-a][1,4]benzodiazepin werden analog Beispiel 8 mit Zn-Staub reduziert.

Nach entsprechender Aufarbeitung erhält man beim Einengen der organischen Phase 0,9 g Öl, das mit Diisopropyläther zur Kristallisation gebracht wird. Die Kristalle werden abgesaugt und getrocknet. Man erhält 400 mg (40 % d.Th.) der Dihydroverbindung vom Fp. 208°C (Zers.).

Beispiel 10

a) 1-Methyl-8-(3-phthalimido-propyloxy)-6-(2-chlorphenyl)-4H-[1,2,4]triazolo[4,3-a][1,4]benzodiazepin

Ein Gemisch von 1,5 g (0,0046 Mol) 8-Hydroxy-1-methyl-6-(2-chlorphenyl)-4H-[1,2,4]triazolo[4,3-a][1,4]benzodiazepin, 1,9 g wasserfreies Kaliumcarbonat und 60 ml Ethylmethylketon wird unter Rühren eine Stunde unter Rückfluß erhitzt. Man läßt die Reaktionsmischung etwas abkühlen, fügt 1,47 g (0,0055 Mol) 3-Brompropylphthalimid zu, erhitzt weitere 7 Stunden zum Sieden und destilliert dann das Lösungsmittel im Vakuum ab. Der Rückstand wird mit Methylenchlorid/$H_2O$ aufgenommen. Die organische Phase wird mit 5%iger Natronlauge und Wasser gewaschen, getrocknet und eingedampft. Der Rückstand wird mit Aceton verrieben; die anfallenden Kristalle werden abgesaugt und säulenchromatographisch an Kieselgel (Methylenchlorid/Methanol 98:2) gereinigt.

Man erhält 1,1 g (46,6 % d.Th.); Fp. 234-236°C der Phthalimidoverbindung.

Analog erhält man:

b) 1-Methyl-8-(2-phthalimido-ethoxy)-6-(2-chlorphenyl)-4H-[1,2,4]triazolo[4,3-a][1,4benzodiazepin.

Fp. 198-199°C (Acetonitril).

Beispiel 11

8-Hydroxy-1-methyl-6-phenyl-4H-[1,2,4]triazolo[4,3-a][1,4]benzodiazepin

Ein Gemisch von 14 g (0,046 Mol) 8-Methoxy-1-methyl-6-phenyl-4H-[1,2,4]triazolo[4,3-a][1,4]benzodiazepin [Fp.197-198°C; Chem. Pharm. Bull. 21, 2382 (1973)] und 150 ml 60%ige Bromwasserstoffsäure wird eineinhalb Stunden unter Rückfluß erhitzt. Die Reaktionslösung wird im Vakuum am Rotationsverdampfer eingeengt, der Rückstand mit Wasser aufgenommen und die Lösung bis zur stark alkalischen Reaktion mit 20%iger Natronlauge versetzt. Man filtriert über Kieselgur und stellt die Reaktionslösung unter Eiskühlung mit 6n Salzsäure auf einen pH von 5 bis 6. Das anfallende Phenol wird nochmals in 2n Natronlauge gelöst und durch Ansäuern (pH 5 bis 6) mit Salzsäure wieder ausgefällt. Die Kristalle werden mit Wasser gewaschen, in Acetonitril suspendiert, abgesaugt und bei 130°C im Vakuumtrockenschrank getrocknet.
Ausbeute: 10 g (70% d.Th.);
Fp. > 320°C (Zers.).

Beispiel 12

8-Hydroxy-1-methyl-6-(2-chlorphenyl)-4H-[1,2,4]triazolo-[4,3-a] [1,4]benzodiazepin

Ausgehend von 8-Methoxy-1-methyl-6-(2-chlorphenyl)-4H-[1,2,4]triazolo[4,3-a][1,4]benzodiazepin (Fp. 199-200°C) erhält man analog Beispiel 11 die Titelverbindung Fp. 302-304°C (Zers.).

$^1$H-NMR ($CD_4O$ TMS int.): δ 2,60 (s, 3H, $CH_3$), 4,29 (d, 1H, $-CH_2-$ A/B System $J_{AB}$ 12 Hz), 5,20 (d, 1H,

-$CH_2$- A/B System, $J_{AB}$ 12 Hz), 6,28 (d, 1H, arom. J 3 Hz), 6,90 (d,d, 1H, arom. J o/m 9 3Hz), 7,28-7,70 (m, 5H, arom.)

Beispiel 13

N-(Ethoxycarbonyl-methyl)-[(1-methyl-6-phenyl-4H-[1,2,4]-triazolo[4,3-a][1,4]benzodiazepin-8-yl)-oxyessigsäureamid]

1,74 g (5mmol)
(1-Methyl-6-phenyl-4H-[1,2,4]triazolo[4,3-a][1,4]-benzodiazepin-8-yl)-oxyessigsäure werden in einem Lösungsmittelgemisch von 40 ml wasserfreiem Tetrahydrofuran und 10 ml wasserfreiem Dimethylformamid vorgelegt. Anschließend gibt man 0,89 g (5,5 mmol) N,N'-Carbonyldiimidazol zu und rührt 2 Stunden bei Raumtemperatur. Dann werden 0,56 g (5,5 mmol) Triethylamin und 0,77 g (5,5 mmol) Glycinethylester-hydrochlorid zugegeben. Nach 15 Stunden wird die Mischung eingeengt, der Rückstand in Dichlormethan/Wasser aufgenommen und nacheinander mit Wasser 2n Natronlauge und Wasser extrahiert. Die organische Phase wird getrocknet, eingeengt und der so erhaltene Rückstand mit Diisopropyläther zur Kristallisation gebracht.
Man erhält 1,5 g (69 % d.Th.) der Titelverbindung vom Schmelzpunkt 152-153°C (Alkohol/Diisopropyläther).

Beispiel 14a

1-Methyl-6-phenyl-8-(3-pyridylmethoxy-4H-[1,2,4]triazolo-[4,3-a][1,4]benzodiazepin

Eine Suspension von 1,74 g (0,006 Mol)
8-Hydroxy-1-methyl-6-phenyl-4H-[1,2,4]triazolo[4,3-a][1.4]-benzodiazepin und 0.6 g Natriumhydrid (50 %ige Suspension

in Mineralöl) in 100 ml wasserfreiem Dimethylformamid wird 90 Minuten unter Stickstoff gerührt und anschließend portionsweise mit 3-Chlormethyl-pyridin-hydrochlorid versetzt. Nach zwölf Stunden wird die Reaktionsmischung am Rotationsverdampfer weitgehend eingeengt, unter Kühlung mit Wasser versetzt und mit Methylenchlorid extrahiert. Die organische Phase wird nacheinander mit 5 %iger Natronlauge und Wasser gewaschen, getrocknet und eingedampft. Den Rückstand reinigt man säulenchromatographisch an Kieselgel [Eluens: Dichlormethan/Methanol (95 : 5)]. Die sauberen Fraktionen werden nach dem Eindampfen aus Acetonitril umkristallisiert.

Ausbeute: 1,1 g (48 % d.Th.); Fp. 199-201°

$C_{23}H_{19}N_5O$ (381.44)

| | | | |
|---|---|---|---|
| Ber.: | C 72.42 | H 5.02 | N 18.36 |
| Gef.: | 72.29 | 5.14 | 18.25 |

Beispiel 14b

1-Methyl-6-phenyl-8-(3-pyridylmethoxy)-4H-imidazo[1,2-a]-[1,4]benzodiazepin

Ausgehend von 1,74 g (0,006 Mol) 8-Hydroxy-1-methyl-6-phenyl-4H-imidazo[1,2-a][1,4]benzodiazepin erhält man in analoger Weise 1,4 g (61,4 % d.Th.) der Titelverbindung.

Schmelzpunkt: 179-180° (Acetonitril)

$C_{24}H_{20}N_4O$ (380,45)

| | | | |
|---|---|---|---|
| Ber.: | C 75.77 | H 5.30 | N 14.73 |
| Gef.: | 75.63 | 5.14 | 14.67 |

Herstellung des Ausgangsmaterials zu Beispiel 14b

8-Methoxy-1-methyl-6-phenyl-4H-imidazo[1,2-a][1,4]benzodiazepin und
8-Hydroxy-1-methyl-6-phenyl-4H-imidazo[1,2-a][1,4]benzodiazepin

Eine Suspension von 35 g (0,124 Mol) 7-Methoxy-6-phenyl-6-phenyl-1H,3H-[1,4]benzodiazepin-2-thion (Fp. 219 - 221°) in 700 ml wasserfreiem Dioxan wird mit 13,7 g Propargylamin versetzt und drei Stunden unter Rückfluß erhitzt. Die Reaktionslösung wird anschließend am Rotationsverdampfer eingeengt und der Rückstand mit Methanol/Wasser aufgenommen. Die organische Phase wird mehrmals mit Wasser gewaschen, über Natriumsulfat getrocknet und eingedampft.

Der Rückstand (35,6 g) wird in 180 ml konzentrierter Schwefelsäure 15 Minuten auf 100° erhitzt. Nach dem Abkühlen gießt man auf Eis, stellt mit Ammoniaklösung alkalisch und extrahiert mit Methylenchlorid. Der nach dem Abdestillieren des Lösungsmittels erhaltene Rückstand wird an einer Kieselgelsäule (Eluens: Methylenchlorid/Methanol 95 : 5 gereinigt.

Die einheitlichen Fraktionen werden vereinigt, eingedampft und umkristallisiert. Man erhält 6,6 g 8-Methoxy-1-methyl-6-phenyl-4H-imidazo[1,2-a][1,4]benzodiazepin vom Schmelzpunkt 184 - 186° (Essigester).

$C_{19}H_{17}N_3O$ (303.37)

| | | | |
|---|---|---|---|
| Ber.: | C 75.23 | H 5.65 | N 13.85 |
| Gef.: | 74.91 | 5.77 | 13.79 |

und 4,9 g 8-Hydroxy-1-methyl-6-phenyl-4H-imidazo-[1,2-a][1,4]benzodiazepin vom Schmelzpunkt 290° Z. (Methanol/Kohle).

$C_{18}H_{15}N_3O$ (289.34)

| | | | |
|---|---|---|---|
| Ber.: | C 74.72 | H 5.23 | N 14.52 |
| Gef.: | 74,59 | 5.27 | 14.38 |

8-Hydroxy-1-methyl-6-phenyl-4H-imidazo[1,2-a][1,4]benzodiazepin kann auch durch Umsetzung von 8-Methoxy-1-methyl-6-phenyl-4H-imidazo[1,2-a][1,4]benzodiazepin mit Bromwasserstoffsäure dargestellt werden.

Beispiel 15a

1-Methyl-8-(3-morpholin-4-yl-propyloxy)-6-phenyl-4H-[1,2,4]-triazolo[4,3-a][1,4]benzodiazepin

Eine Suspension von 5 g (0,0172 Mol) 8-Hydroxy-1-methyl-6-phenyl-4H-[1,2,4]triazolo[4,3-a][1,4]-benzodiazepin und 9,5 g wasserfreies Kaliumcarbonat in 200 ml Ethylmethylketon wird eine Stunde unter Rühren auf Rückflußtemperatur erhitzt. Nach dem Abkühlen wird die Reaktionsmischung mit 3,78 g N-(3-Chlorpropyl)-morpholin-hydrochlorid versetzt und 18 weitere Stunden unter Rückfluß erhitzt. Anschließend wird das Lösungsmittel abdestilliert und der Rückstand in Methylenchlorid/Wasser aufgenommen. Die organische Phase wird nacheinander mit verdünnter Natronlauge und Wasser gewaschen, getrocknet und eingedampft. Das zurückbleibende Rohprodukt wird säulenchromatografisch gereinigt (Kieselgel; Methylenchlorid/Methanol 95 : 5).

Ausbeute: 4,4 g (61 % d.Th.); Fp. 145 - 147°

$C_{24}H_{27}N_5O_2$ (417.51)

| | | | |
|---|---|---|---|
| Ber.: | C 69.04 | H 6.52 | N 16.77 |
| Gef.: | 68.84 | 6.60 | 16.55 |

Beispiel 15b

Analog zu Beispiel 15a erhält man

1-Methyl-8-(3-morpholino-propyloxy)-6-phenyl-4H-imidazo-[1,2-a][1,4]benzodiazepin

durch Umsetzung von
8-Hydroxy-1-methyl-6-phenyl-4H-imidazo[1,2-a][1,4]benzodiazepin mit N-(3-Chlorpropyl)-morpholin-hydrochlorid

Schmelzpunkt: 133 - 134° (Essigester/Diisopropyläther)

$C_{25}H_{28}N_4O_2$ (416.53)

| | | | |
|---|---|---|---|
| Ber.: | C 72.09 | H 6.78 | N 13.45 |
| Gef.: | 72.18 | 6.62 | 13.39 |

Weitere erfindungsgemäße Verbindungen sind in den nachstehenden Tabellen aufgeführt:

TABELLE I

Verbindungen der Formel

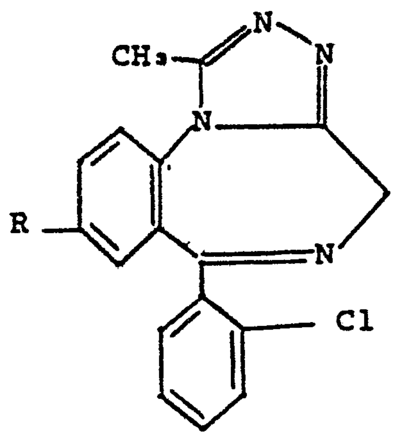

| Nr. | R | Fp. | Bemerkungen |
|---|---|---|---|
| 1 | 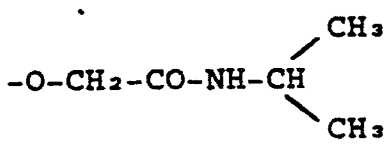 | | |
| 2 | 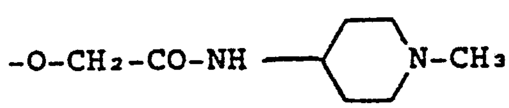 | | |
| 3 | 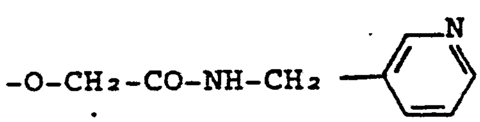 | 162-165°C | |
| 4 | 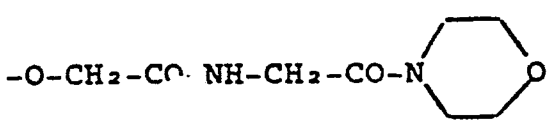 | | |
| 5 | $-O-CH_2-CO-NH-(CH_2)_2-OH$ | | |
| 6 | 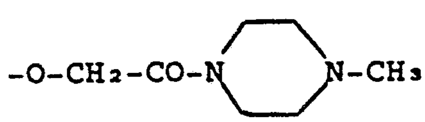 | 215-216°C | |

| Nr. | R | Fp. | Bemerkungen |
|---|---|---|---|
| 7 | 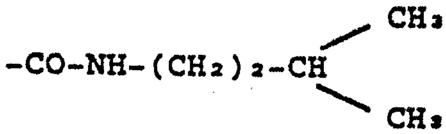 | 153-155°C | |
| 8 | $-O-CH_2-CO-NH_2$ | | |
| 9 | 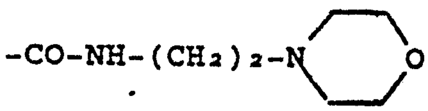 | 239-240°C | |
| 10 | $-O-CH_2-CO-CH_2-COOEt$ | | |
| 11 | 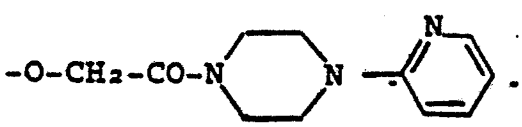 | 215-216°C | |
| 12 | 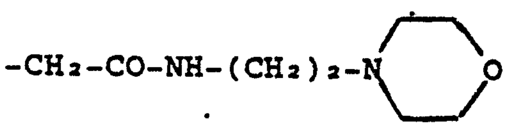 | | |
| 13 | 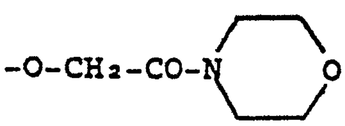 | | |
| 14 | 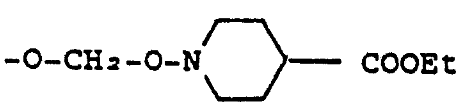 | | |
| 15 | $-O-CH_2-CO-N(Et)_2$ | | |
| 16 | 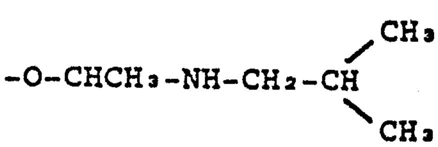 | 215-217°C | |

| Nr. | R | Fp. | Bemer-kungen |
|---|---|---|---|
| 17 | 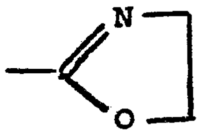 | 229-231°C | |
| 18 | 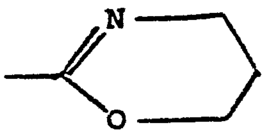 | 153-155°C | |
| 19 | $-CO-O-(CH_2)_3-N(CH_3)_2$ | 133-135°C | |
| 20 | $-CO-O-(CH_2)_3-N(CH_3)_3{}^+J^-$ | 260-261°C | |
| 21 | -CO-O- 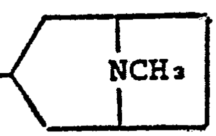 | 283-285°C | |
| 22 | $-CO-O-CH(CH_3)-CH_2-N(C_2H_5)_2$ | 137-139°C | |
| 23 | 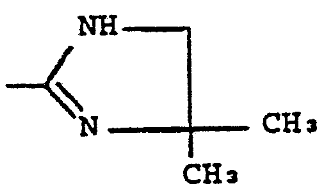 | | |

TABELLE II

Verbindungen der Formel Ia

| Nr. | A | B | R | $R_1$ | $R_2$ | Fp. |
|---|---|---|---|---|---|---|
| 1 | CH | N | 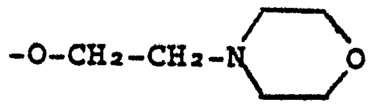 | $CH_3$ | $C_6H_5$ | |
| 2 | N | N | 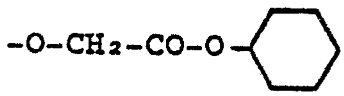 | $CH_3$ | $C_6H_5$ | |
| 3 | N | CH | 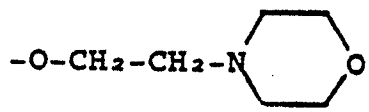 | $CH_3$ | $C_6H_5$ | |
| 4 | CH | N | 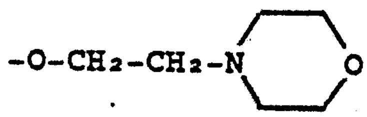 | H | $C_6H_5$ | |
| 5 | N | N | 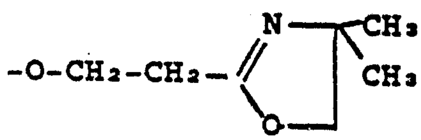 | $CH_3$ | $C_6H_5$ | |
| 6 | N | N | 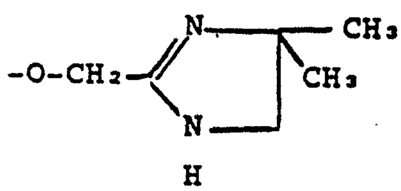 | $CH_3$ | $C_6H_5$ | |
| 7 | N | N | 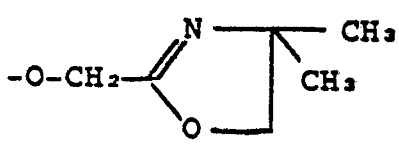 | $CH_3$ | $C_6H_5$ | |
| 8 | N | CH | 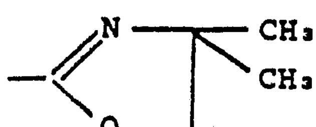 | $CH_3$ | $2ClC_6H_4$ | |
| 9 | N | N | 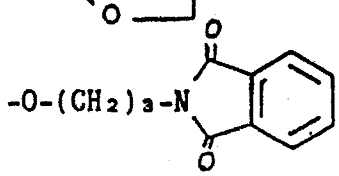 | $CH_3$ | $C_6H_5$ | 187-188° |
| 10 | N | N | 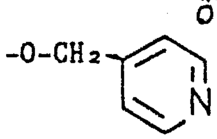 | $CH_3$ | $C_6H_5$ | 194-196° |

NMR-Spektren zu Verbindungen der TABELLE I ($\delta$ in ppm)

Zu Nr. 1

$^1$H-NMR ($CDCl_3$, TMS int.) $\delta$ 0,90 (d, 6H, $2CH_3$, J 7Hz), 1,79 (m, 1H, CH), 2,61 (s, 3H, $CH_3$), 3,16 (d,d, 2H, $-CH_2-$ J 6Hz), 4,17 (d, 1H, $-CH_2-$ A/B System $J_{AB}$ 12 Hz), 4,45 (s, 2H, $-CH_2-$), 5,56 (d, 1H, A/B System $-CH_2-$, $J_{AB}$ 12 Hz), 6,50 (d, 1H, NH), 6,75 (d, 1H arom. J 3 Hz), 7,11-7,72 (m, 6H, arom.).

Zu Nr. 2

$^1$H-NMR ($CDCl_3$, TMS int.) $\delta$ 1,22-2,43 (m, 6H, Piperidin), 2,28 (s, 3H, $>NCH_3$), 2,60 (s, 3H, $CH_3$), 2,80 (m, 2H, Piperidin), 3,84 (m, 1H, Piperidin), 4,15 (d, 1H, $-CH_2-$ A/B System $J_{AB}$ 12 Hz), 4,37 (s, 2H, $-CH_2-$), 5,50 (d, 1H, $-CH_2-$ A/B System $J_{AB}$ 12 Hz), 6,36 (d, 1H, >NH), 6,71 (d, 1H arom. J 3 Hz), 7,10-7,70 (m, 6H, arom.).

Zu Nr. 4

$^1$H-NMR ($CDCl_3$, TMS int.) $\delta$ 2,61(s, 3H, $CH_3$), 3,31-3,84 (m, 8H, Morpholin), 4,13 (d, 2H, $-CH_2-$), 4,17 (d, 1H, $-CH_2-$ $J_{AB}$ 12 Hz), 4,47 (s, 2H, $-CH_2-$), 5,52 (d, 1H, $-CH_2-$ A/B System $J_{AB}$ 12 Hz), 6,75 (d, 1H, arom. J 3Hz), 7,13-7,70 (m, 6H, arom.).

Zu Nr. 5

$^1$H-NMR ($CDCl_3$, TMS int.) $\delta$ 2,56 (s, 3H, $CH_3$), 3,52 (m, 2H, $-CH_2-$), 3,75 (t, 2H, $-CH_2-$), 4,15 (d, 1H, $-CH_2-$ A/B Sytem $J_{AB}$ 12 Hz), 4,45 (s, 2H, $-CH_2-$), 5,50 (d, 1H, $-CH_2-$ A/B System $J_{AB}$ 12 Hz), 6,72 (d, 1H, arom. J 3 Hz), 7,00-7,70 (m, 6H, arom, 1>NH).

Zu Nr. 8

$^{1}$H-NMR ($CDCl_3$, TMS int.) δ 2,59 (s, 3H, $CH_3$), 4,17 (d, 1H, $-CH_2-$ A/B System $J_{AB}$ 12 Hz), 4,45 (s, 2H, $-CH_2-$), 5,52 (d, 1H, $-CH_2-$ A/B System $J_{AB}$ 12 Hz), 5,93, 6,52 (1H/1H, >$NH_2$), 6,75 (d, 1H, arom. J 3Hz), 7,10 (m, 6H arom.)

Zu Nr. 10

$^{1}$H-NMR ($CDCl_3$, TMS int.) δ 1,29 (t, 3H, $CH_3$, J 7Hz), 2,61 (s. 3H, $CH_3$), 4,11 (d, 2H, $-CH_2-$, J 5Hz), 4,18 (d, 1H, $-CH_2-$ A/B System $J_{AB}$ 12 Hz), 4,25 (q, 2H, $-CH_2-O-$), 4,50 (s, 2H, $-CH_2-$), 5,52 (d, 1H, $-CH_2-$ A/B System $J_{AB}$ 12 Hz), 6.75 (d, 1H arom. J 3Hz), 7,09 (t, 1H, >NH), 7,13-7,70 (m, 6H, arom.).

Zu Nr. 12

$^{1}$H-NMR ($CDCl_3$, TMS int.) δ 2,45 (m, 4H, Morpholin), 2,52 (t, 2H, $-CH_2-$), 2,61 (s, 3H, $CH_3$), 3,43 (m, 2H, $-CH_2-$), 3,63 (m, 4H, Morpholin), 4,18 (d, 1H, $-CH_2-$ A/B System $J_{AB}$ 12 Hz), 4,45 (s, 2H, $-CH_2-$), 5,54 (d, 1H, $-CH_2-$ A/B System $J_{AB}$ 12 Hz), 6,73 (d, 1H, arom., J 3Hz), 7,00-7,72 (m, 6H, arom. 1 >NH).

Zu Nr. 13

$^{1}$H-NMR ($CDCl_3$, TMS int.) δ 2,59 (s, 3H, $CH_3$), 3,36-3,79 (m, 8H, $-CH_2-$ Morpholin), 4,17 (d, 1H, $-CH_2-$, A/B System $J_{AB}$ 12 Hz), 4,67 (s, 2H, $-CH_2-$), 5,51 (d, 1H, $-CH_2-$ A/B System $J_{AB}$ 12 Hz), 6,75 (d, 1H, arom. J 3 Hz), 7,11-7,70 (m, 6H, arom.).

Zu Nr. 14

$^1$H-NMR ($CDCl_3$, TMS int.) δ 1,27 (t, 3H, $CH_3$ J 7Hz), 1,43-2,11 (m, 4H, Piperidin), 2,34-3,45 (m, 3H, Piperidin), 2,61 (s, 3H, $CH_3$), 3,61-4,43 (m, 2H, Piperidin), 4,18 (q, 2H, $-CH_2-O-$), 4,18 (d, 1H, $-CH_2-$ A/B System $J_{AB}$ 12 Hz), 4,65 (s, 2H, $-CH_2-$), 5,52 (d, 1H, $-CH_2-$ A/B System $J_{AB}$ 12 Hz), 6,75 (d, 1H, arom. J 3Hz), 7,11-7,68 (m, 6H, arom.).

Zu Nr. 15

$^1$H-NMR ($CDCl_3$, TMS int.) δ 1,09, 1,13 (t, 6H, $CH_3$), 2,60 (s, 3H, $CH_3$), 3,31 (m, 4H, $CH_2-N-$), 4,18 (d, 1H, $-CH_2-$ A/B System $J_{AB}$ 12 Hz), 4,65 (s, 2H, $-CH_2-$), 5,50 (d, 1H, $-CH_2-$ A/B System $J_{AB}$ 12 Hz), 6,72 (d, 1H, arom. J 3 Hz), 7,10-7,65 (m, 6H, arom.).

NMR-Spektren zu Verbindungen der Tabelle II (δ in ppm)

Nr. 1

$^1$H-NMR ($CDCl_3$, TMS int.) δ 2.36 (d, 3H, J = <2Hz), 2.54 (m, 4H, $N-CH_2$-Morpholin), 2.77 (t, 2H, J = 6Hz, $N-CH_2-CH_2O$); 3.72 (m, 4H, $OCH_2$-Morpholin), 4.02 (d, 1H, $-CH_2$A/B-System, $J_{AB}$ 12Hz), 4.08 (t, 2H, J = 6Hz, $O-CH_2-CH_2N$), 5.26 (d, 1H, $-CH_2$-A/B-System, $J_{AB}$ 12Hz), 6.87 (qu., 1H, J = <2Hz, CH=), 6.91 - 7.78 (m, 8H, arom.).

Nr. 6

$^1$H-NMR ($CDCl_3$, TMS int.) δ 1.24 [s, 6H $(CH_3)_2-C$)], 2.63 (s, 3H, $CH_3$), 3.38 (s, 3H, $N-CH_2$, NH), 4.08 (d, 1H, $-CH_2$-A/B-System, $J_{AB}$ 13 Hz), 4.67 (s, 2H, $OCH_2$), 5.44 (d, 1H-$CH_2$-A/B-System, $J_{AB}$ 13Hz) 6.85 - 7.71 (m, 8H, arom.).

## Patentansprüche

1. Verbindungen der Formeln

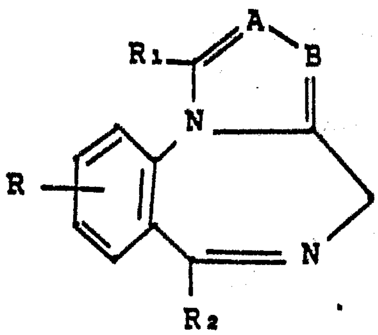

(Ia)

oder

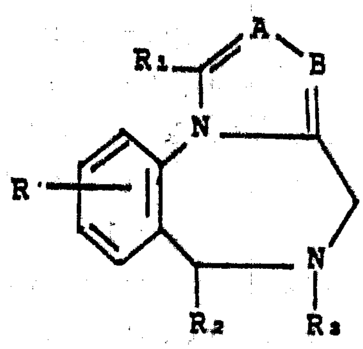

(Ib),

worin

R für eine der Gruppen

(a) $-O-X-CO-OR_4$

(b) $-O-X-CO-R_5$

(c) $-O-Y-R_6$

(d) $-Z-CO-OR_4$

(e) $-Z-CO-R_5$

(f) 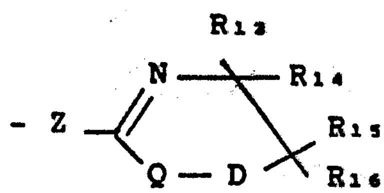 ;

(g) 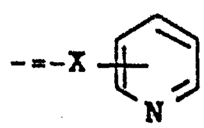

$R_1$ für Wasserstoff; für $C_1$-$C_4$-Alkyl oder für $C_3$-$C_6$-Cycloalkyl; oder, falls R die Gruppe (d), (e) oder (f) bedeutet, auch für $C_1$-$C_4$-Alkoxy oder Halogen;

$R_2$ für Phenyl, für ein- oder mehrfach durch Methyl, Halogen, Nitro, Trifluormethyl oder, falls R die Gruppe (d), (e) oder (f) bedeutet, auch durch Methoxy substituiertes Phenyl; oder für α-Pyridyl;

$R_3$ für Wasserstoff oder für $C_1$-$C_4$-Alkyl;

$R_4$ für einen durch eine Mono- oder Di-($C_1$-$C_3$-alkyl)aminogruppe substituierte $C_1$-$C_6$-Alkylgruppe; für $C_3$-$C_7$-Cycloalkyl, das im Falle des 5- oder 6-Rings auch eine >N($C_1$-$C_4$-Alkyl)-Gruppe als Ringglied enthalten kann und das im Fall des $C_7$-Cycloalkyls auch eine >$NCH_3$-Brücke aufweisen kann; oder, falls R die Gruppe (a) oder (d) bedeutet, auch für Wasserstoff oder $C_1$-$C_6$-Alkyl;

$R_5$ für eine der Gruppen

(a) 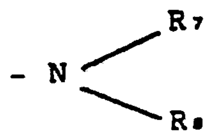 oder

(b) 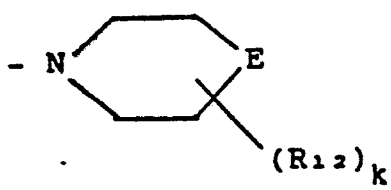 ;

$R_6$ für eine der Gruppen

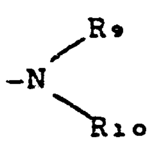 oder 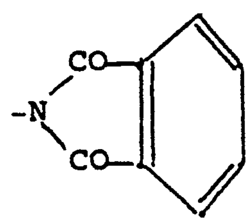 oder 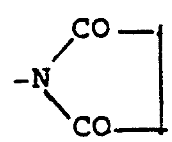 ;

oder die unter R (f) aufgeführte Gruppe, worin Z eine Einfachbindung darstellt;

$R_7$ und $R_8$ (unabhängig voneinander) für Wasserstoff; für $C_1$-$C_6$-Alkyl, Allyl oder Propargyl; und, falls $R_7$ Wasserstoff oder Methyl bedeutet, $R_8$ auch für $C_3$-$C_6$-Cycloalkyl, Aryl, Aralkyl oder eine der Gruppen

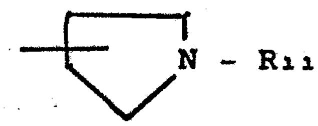

oder 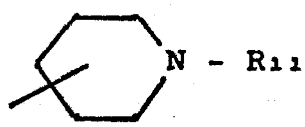

oder 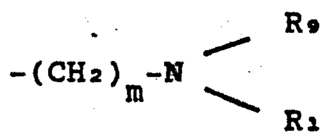 ;

oder,
falls R die Gruppe (b), darstellt, auch für eine der Gruppen

$-CHR_3-COO(C_1-C_4$-Alkyl) oder

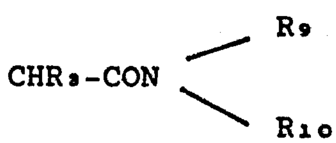 ;

$R_9$ und $R_{10}$ (unabhängig voneinander) für Wasserstoff; für $C_1$-$C_6$-Alkyl; außerdem (zusammen mit dem Stickstoffatom der Gruppe $-NR_9R_{10}$) für einen gegebenenfalls ein- bis vierfach methylsubstituierten gesättigten fünf- oder einen sechsgliedrigen Ring, wobei dieser auch ein weiteres Heteroatom (O, S, $NR_3$) enthalten kann;

$R_{11}$ für $C_1$-$C_4$-Alkyl, Aryl oder Aralkyl;

$R_{12}$ für Wasserstoff oder Methyl, einer der Reste $R_{12}$ gegebenenfalls auch für -CO-O($C_1$-$C_6$-Alkyl),

$R_{13}$ für Wasserstoff; für ein gegebenenfalls hydroxy- oder aminosubstituiertes $C_1$-$C_6$-Alkyl; für eine CO-O($C_1$-$C_4$-Alkyl)- oder eine CO-N($C_1$-$C_4$-Alkyl)$_2$-Gruppe;

$R_{14}$, $R_{15}$, $R_{16}$, $R_{17}$ und $R_{18}$ (unabhängig voneinander) für Wasserstoff; für ein gegebenenfalls hydroxy- oder aminosubstituiertes $C_1$-$C_6$-Alkyl; oder für Phenyl;

A und B (unabhängig voneinander) für N, CH oder C-$CH_3$ aber nicht beide für CH oder C-$CH_3$;

D für $(CR_{17}R_{18})_n$;

Q für O, S, NH oder N($C_1$-$C_6$-Alkyl);

E für O, S, $NR_{11}$, $CH_2$ oder eine Einfachbindung;

X für $C_1$-$C_6$-Alkylen;

Y für $C_2$-$C_6$-Alkylen;

Z für $C_1$-$C_6$-Alkylen oder eine Einfachbindung;

k für 1, 2, 3 oder 4;

m für 2 oder 3;

n für O, 1 oder 2

stehen kann, gegebenenfalls in Form der Racemate, der Enantiomeren, Diastereomeren und ihrer Gemische; jeweils als freie Basen oder als Säureadditionssalze, gegebenenfalls auch als quaternisierte Verbindungen.

2. Verbindungen der allgemeinen Formeln Ia oder Ib nach Anspruch 1, worin A und B Stickstoff oder A CH-Gruppe und B Stickstoff;

$R_1$: Methyl, Methoxy und Cyclopropyl;

$R_2$: 2-Chlorphenyl;

R: in 8-Stellung mit dem Ringsystem verknüpft, wobei für die Gruppen (a) bis (g) gilt

bei (a): X gleich $C_1$-$C_3$-Alkylen
$R_4$ in der obigen Bedeutung, ausgenommen Wasserstoff und $C_1$-$C_6$-Alkyl;

bei (b): X gleich $C_1$-$C_3$-Alkylen,
$R_5$ gleich $-NR_7R_8$ mit $R_7$, $R_8$ gleich Wasserstoff oder $C_1$-$C_4$-Alkyl;
oder
$R_7$ gleich H und
$R_8$ gleich $C_2$-$C_3$-Alkylsubstituiert durch N-Morpholino oder 2,6-Dimethylmorpholino;
oder

$R_5$ gleich 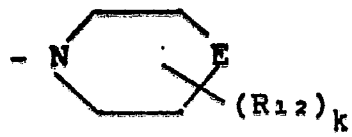 ,

worin
E gleich O, N-Methyl, $CH_2$;
dabei ist im Falle E gleich O, $R_{12}$ bevorzugt Methyl und k gleich 2, wobei die Methylgruppen sich in 2- und 6-Stellung befinden, während für E gleich $CH_2$ $R_{12}$ vor allem $COOC_2H_5$ ist;

bei (c): Y gleich $C_1$-$C_3$-Alkylen,
$R_6$ Phthalimido oder $NR_9R_{10}$ und $R_9/R_{10}$ gleich H oder $C_1$-$C_4$-Alkyl oder $NR_9R_{10}$ gleich Morpholino, gegebenenfalls methylsubstituiert;

bei (d): Z eine Einfachbindung,
$R_4$ in der obigen Bedeutung, ausgenommen Wasserstoff und $C_1$-$C_6$-Alkyl;

bei (e): Z eine Einfachbindung,
$R_5$ $NR_7R_8$ mit $R_7$ gleich H,

$R_8$ $-CH_2-CH_2-$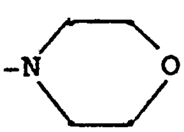 oder

$-C_1-C_6$-Alkyl;

bei (f) Z eine Einfachbindung, ebenso D (n = O), $R_{13}/R_{14}$ Methyl, $R_{15}/R_{16}$ Wasserstoff,

bei (g) Z $-CH_2-$ oder $-CH_2-CH_2-$

bedeuten kann, gegebenenfalls in Form ihrer Racemate, ihrer Entantiomeren, ihrer Diastereomeren und ihrer Gemische; jeweils als freie Basen oder als Säureadditionsalze, gegebenenfalls auch als quaternisierte Verbindungen

3. Pharmazeutische Zubereitungen, gekennzeichnet durch einen Gehalt an einer Verbindung nach Anspruch 1 oder 2 neben üblichen Träger- und/oder Hilfsstoffen.

4. Verwendung von Verbindungen nach Anspruch 1 oder 2 bei der Behandlung von Krankheiten, an denen PAF beteiligt ist.

5. Verbindungen nach Anspruch 1 oder 2 zur Verwendung für die Herstellung von pharmazeutischen Zubereitungen mit PAF-antagonistischer Wirkung.

6. Methode zur Behandlung von Erkrankungen, an denen der PAF beteiligt ist, dadurch gekennzeichnet, daß man eine Verbindung nach Anspruch 1 oder 2 verwendet.

7. Verfahren zur Herstellung von Verbindungen gemäß Anspruch 1 oder 2 nach üblichen Methoden, dadurch gekennzeichnet, daß man

a) eine Verbindung der Formel II mit einer Verbindung der Formel III umsetzt
oder daß man

b) eine Verbindung der Formel Ia, in der R für -O-X-COOH oder -Z-COOH bzw. ein reaktionsfähiges Derivat davon steht, mit Aminen der Formel VI oder VII oder mit Ammoniak umsetzt
oder daß man

c) eine Verbindung der Formel II mit einer Verbindung der Formel VIII umsetzt
oder daß man

d) eine Verbindung der Formel Ia, in der R für $-O-Y-R'_6$ steht, worin $R'_6$ die Phthalimidogruppe bedeutet, einer Hydrazinolyse unterwirft.
oder daß man

e) eine Verbindung der Formel X mit einem bisfunktionalisierten Amin umsetzt
oder daß man

f) eine Verbindung der Formel Ia, in der R eine hydroxyfunktionalisierte Carbonsäureamidgruppe bedeutet, einem Ringschluß mit Thionylchlorid unterwirft und den Ringsauerstoff gewünschtenfalls anschließend gegen Schwefel austauscht
oder daß man

g) eine Verbindung der Formel Ia, worin R für -Z-CN oder -O-Y-CN steht, in das Imidoesterhydrochlorid umwandelt und dieses mit einem entsprechenden Diamin umsetzt
oder daß man

h) eine Verbindung der Formel Ia, in der $R_1$ H bedeutet, zu einer Verbindung mit $R_1$ gleich Halogen halogeniert
oder daß man

i) eine Verbindung der Formel Ia, in der $R_1$ Brom oder Chlor mit einem Alkoholat umsetzt oder daß man

k) eine Verbindung der allgemeinen Formel II in Gegenwart einer Base oder als Anion mit einer Verbindung der allgemeinen Formel

Halogen - X 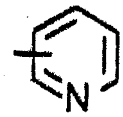

umsetzt
oder daß man

l) eine Verbindung der Formel Ia mit üblichen Mitteln zu entsprechenden Verbindungen Ib mit $R_3$ gleich H reduziert und gewünschtenfalls diese Verbindungen zu Verbindungen mit $R_3$ gleich Alkyl alkyliert
oder daß man

m) funktionalisierte Verbindungen aus Verbindungen der Formel Ib mit R gleich einem Rest der Gruppe (c) mit entsprechenden Reaktionspartnern umsetzt
oder daß man

n) den ankondensierten Imidazolo- oder Triazoloring durch Ringschlußreaktion erzeugt

und daß man gewünschtenfalls erhaltene Produkte in etwa vorliegende Enantiomere oder in diastereomere Antipodenpaare auftrennt und/oder daß man zunächst erhaltene Säureadditionssalze in freie Basen, zunächst erhaltene Basen in Säureadditionssalze überführt